# EUROPEAN PATENT APPLICATION

(11) **EP 3 381 897 A1**
(43) Date of publication of application: **03.10.2018**
(21) Application number: 17163018.9
(22) Date of filing: 27.03.2017
(51) Int. Cl.: C07D 207/335, A61K 31/40, A61P 35/00

(54) **DERIVATIVES OF THE DISODIUM 2,2'-{CARBONYLBIS[IMINO-3,1-PHENYLENECARBONYLIMINO(1-METHYL-1H-PYRROLE-4,2-DIYL)CARBONYLIMINO]}DINAPHTHALENE-1,5-DISULFONATE SALT AND RELATED COMPOUNDS AS HEPARANASE INHIBITORS FOR THE TREATMENT OF CANCER**

(71) Applicant: Leadiant Biosciences SA, 6850 Mendrisio (CH)
(72) Inventor: GIANNINI, Giuseppe, 00071 Pomezia (ROMA) (IT); SIMONI, Daniele, 44123 Ferrara (IT); SEMERARO, Floriana, 36075 Montecchio Maggiore (VI) (IT); RONDANIN, Riccardo, 45023 Costa di Rovigo (RO) (IT)
(74) Representative: Ghirardi, Valeria

(57) **Abstract**

The present invention relates to ureido derivatives of naphtalenesulfonic acids having an anti-heparanase activity.
In particular, it relates to compounds of formula (I) 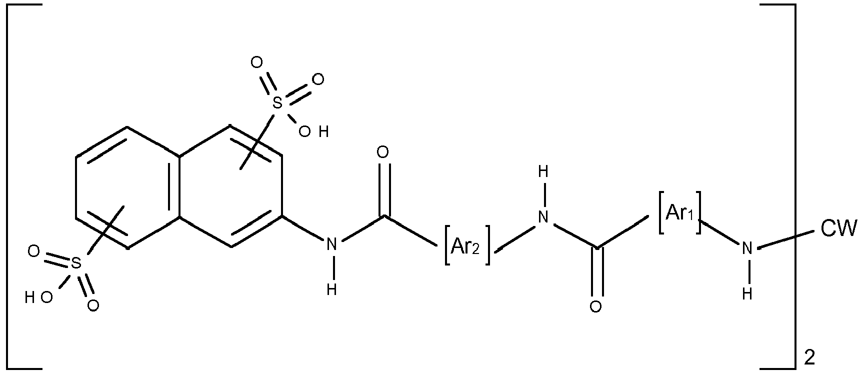 for use in the treatment of diseases where an inhibition of heparanase is desired, in particular cancer.
The present invention also relates in particular to derivatives of the disodium 2,2'-{carbonylbis[imino-3,1-phenylenecarbonylimino(1-methyl-1H-pyrrole-4,2-diyl)carbonylimino]}dinaphthalene-1,5-disulfonate salt and related compounds.

## Description

### FIELD OF THE INVENTION

The present invention relates to ureido derivatives of naphtalenesulfonic acids having an anti-heparanase activity.

The invention also relates to the use of such compounds as a medicament, in particular for the treatment of diseases where an inhibition of heparanase is desired, and to pharmaceutical compositions comprising the same.

### BACKGROUND OF THE INVENTION

Heparan sulfate proteoglycans (HSPGs) are ubiquitous macromolecules associated with the cell surface and extracellular matrix (ECM) of a wide range of cells of vertebrate and invertebrate tissues (Bernfield et al. Annu. Rev. Biochem. 1999, 68, 729; lozzo R. V., San Antonio J. D. J. Clin. Invest. 2001, 108, 349; Kjellen L., Lindahl U. Annu. Rev. Biochem. 1991, 60, 443). The basic HSPG structure consists of a protein core to which several linear heparan sulfate (HS) glycosaminoglycan side chains are covalently O-linked.

HS glycosaminoglycans bind to and assemble a multitude of ECM proteins (i.e., laminin, fibronectin, collagen type IV) and thereby contribute significantly to the ECM self-assembly and integrity. Moreover, the HS chains ensure that a wide variety of bioactive molecules bind to the cell surface and ECM and thereby function in the control of normal and pathological processes, among which are morphogenesis, tissue repair, inflammation, vascularization and cancer metastasis.

Heparanase, an endoglucuronidase responsible for heparan sulfate (HS) cleavage, regulates the structure and function of heparan sulfate proteoglycans, thus resulting in structural alterations of the ECM and release of bioactive saccharide fragments and HS-bound growth factors and cytokines. There is growing evidence that heparanase upregulates expression of genes that participate in cancer metastasis and angiogenesis, glucose metabolism, immune response, inflammation and atherosclerosis, suggesting that heparanase belongs to an emerging class of proteins that play a significant role in regulating transcription in addition to their well-recognized extra-nuclear functions (Pisano C. et al. Biochem. Pharmacol. 2014, 89, 12; Ilan N. et al. Int. J. Biochem. Cell Biol. 2006, 38, 2018; Fux L. et al. Trends Biochem. Sci. 2009, 34, 511; Parish C.R.et al. Matrix Biol. 2013, 32, 228; Ramani V.C. et al. FEBS J. 2013, 280, 2294; Vlodavsky I. et al. Matrix Biol. 2013. 32, 241; Yang Y. et al. Cancer Res. 2010, 70, 8329).

In recent years, heparanase has attracted considerable attention as a promising target for innovative pharmacological applications. Indeed, heparanase appears to be involved in major human diseases, from tumors to chronic inflammation, diabetic nephropathy, bone osteolysis, thrombosis and atherosclerosis, in addition to more recent investigation in various rare diseases (Future Med Chem. 2016 Apr;8(6):647-80. Heparanase: a rainbow pharmacological target associated to multiple pathologies including rare diseases. Rivara S, Milazzo FM, Giannini G).

In view of the above, compounds able to specifically modulate the activity of this enzyme are highly desired as a useful pharmacological option for many therapeutic indications. Since heparanase is involved in a very wide range of molecular pathways, modulation of the expression or activity of this enzyme is a viable therapeutic option. Potent and selective heparanase inhibitors are therefore highly searched as therapeutic tools for those clinical indications in which heparanase inhibition is pharmacologically useful.

However, no drug able to inhibit or modulate heparanase functions has yet been registered (Rivara S, 2016).

Some compounds able to inhibit heparanase are known in the art.

In particular, three classes of heparanase inhibitors are known: active analogous of endogenous substance, synthetic small molecule compounds and natural products (see some review: Ferro V. Mini Rev. Med. Chem. 2004, 4, 693; Jia L., Ma S. Eur. J. Med. Chem. 2016, 121, 209; Rivara S. et al. Future Med. Chem. 2016, 8, 647).

As one of the closest mimics of HS, heparin is a natural choice as a heparanase inhibitor. However, it is limited as an anti-cancer drug due to its potent anticoagulant activity (Casu B. et al. Pathophysiol. Haemost. Thromb. 2008, 36, 195).

Among small molecules, suramin (Scheme 1), a polysulfonated naphthylurea with antiangiogenic and anti-proliferative activity, was found to inhibit heparanase (Nakajima M. et al. J. Biol. Chem. 1991, 266, 9661). Suramin has undergone extensive clinical evaluation as inhibitor of growth factors bFGF, TNFα, VEGF and IL-8, as well as anticancer agent.

However, suramin has found so far a limited clinical usefulness because of severe toxicity associated with indiscriminate inhibition of many other enzymes.

The suramin analogues chosen because they possess higher antiangiogenic activity than suramin itself were also potent heparanase inhibitors (IC₅₀ = 20-30 µM) (Marchetti D. et al. Int. J. Cancer 2003, 104, 167).

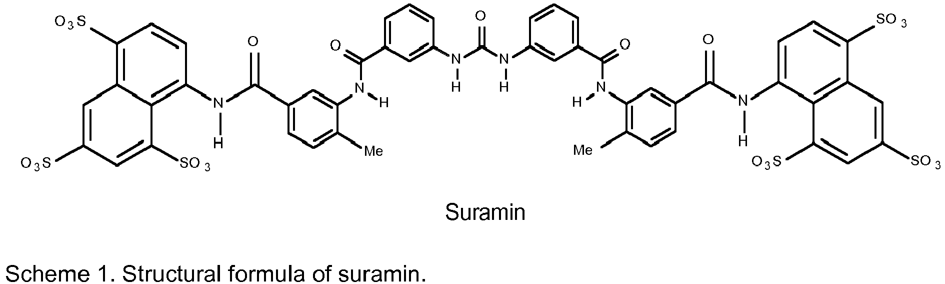

Besides studies on suramin, related sulfonated distamycin-A derivatives, sometimes reported as suradistas, were prepared and studied (Ciomei M. et al. Biochem. Pharmacol. 1994, 47, 295; Sola F. et al. Invasion Metastasis 1995, 15, 222; Finch P. W. Pharmacology, 1997, 55, 269; Manetti F. et al. Bioorg. Med. Chem. 1998, 6, 947; Schneider G. P. et al. Clin. Cancer Res. 2002, 8, 3955). In this series, compounds as FCE 27266 demonstrated the same promising pattern of antimetastatic and antiangiogenic action as suramin, but less cytotoxicity. The antimetastatic and antiangiogenic activity of FCE 27266 have been related to its ability to complex and inhibit several growth factors (bFGF, PDGFb, VEGF) as well as proteinases enzymes of the extracellular matrix which cover a central role in the angiogenesis and metastatic processes (see also Lozano R. M. J. Mol. Biol. 1998, 281, 899). It was also suggested that FCE27266 antimetastatic activity could be due to inhibition of matrix-degrading enzymes released by tumor and endothelial cells during the process of metastatic spread and neovascularization (Sola, F; Farao, M; Ciomei, M; Pastori, A. Mongelli, N. et al. Invasion Metastasis. 1995, 15, 222-31). It has also been shown that FCE27266 inhibits binding of bFGF to its receptor and this mechanism has been proposed as responsible of its anti-angiogenetic activity (Manetti F., Botta M., Mongelli N. et al. Bioorg. Med. Chem. 1998, 947).

Further derivatives of dystamicin A with different activities and therapeutic uses have been disclosed and studied.

For example, WO9110649, GB2261661 and GB2260134 disclose pyrrole derivatives of dystamicin A as angiogenesis inhibitors.

WO9420095 discloses the use of a selected class of such compounds as antimetastatic agents. WO9423718 discloses the use of said compounds in the treatment of lentivirus-induced diseases while WO9728796 discloses their use in the treatment of multiple sclerosis. WO9523806 discloses a further selected class of the above mentioned pyrrole derivatives.

WO9605196 discloses dystamicin A derivatives as antitumor or antiviral agents.

WO9626950 discloses polypirrolcarboxamidonapthalene derivatives for improving bioavailability of an active compound.

WO2015008138 discloses ureido derivatives of napthalenesulfonic acids for the treatment of HIV infections.

However, none of the above mentioned prior art documents discloses an anti-heparanase activity of the disclosed compounds.

Compounds having a potent anti-heparanase activity have now been found.

In particular, it has now been found that the known compound FCE27266 has a potent anti-heparanase activity, in particular about 20 times more than suramin (known as a potent heparanase inhibitor).

Indeed, it has been found that FCE27266 has values of heparanase inhibitory activity at low micromolar level (IC₅₀= 2.0 µM) in the commonly used AGAIA test (Hammond et al. Anal. Biochem. 2010, 396, 112). In view of the fact that the IC50 inhibitory activity of suramin is about 26 µM, while the most active analogues of suramin do not reach the values of 20µM, such result indicates a very surprising potent heparanase inhibitory activity of FCE27266.

It has also been found that new derivatives of FCE27266 wherein a pyrrole ring has been replaced with a benzene ring are inhibitors of heparanase more powerful than suramin and its analogues and also more active than the same FCE27266. Furthermore, such new compounds advantageously present a lower toxicity with respect to suramin and this render them more suitable to clinical applications as heparanase inhibitors.

Furthermore, it has been found that the new compounds of the invention are able to inhibit invasion of tumor cells, such as human fibrosarcoma cells, therefore providing a basis for their application in the inhibition of tumor growth.

### SUMMARY OF THE INVENTION

It is an object of the present invention a compound having the following formula (I) 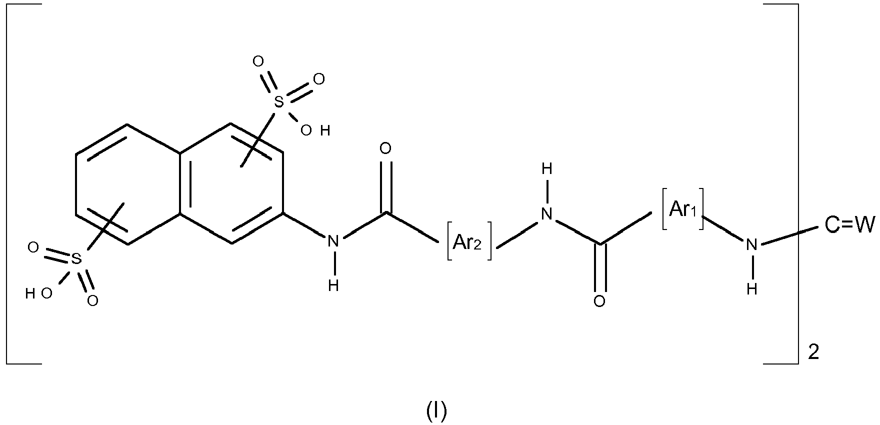 wherein
Ar₁ and Ar₂ can be the same or different and independently selected from the group consisting of: 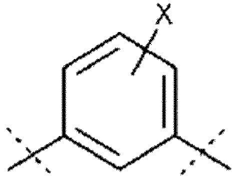
wherein X is selected from the group consisting of H, C₁-C₄alkyl, linear or branched, halogen and C₁-C₄alkyl substituted by one or more halogens;
and 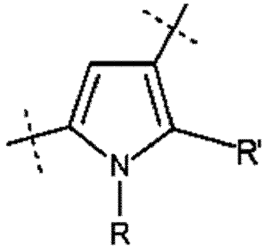
wherein each of R and R' is independently selected from the group consisting of H, C₁-C₄ alkyl, linear or branched, C₁-C₄alkyl substituted with one or more halogens, and cyclopropyl;
and W is selected from the group consisting of O, S and NH,
the enantiomers, diastereoisomers, and mixtures thereof,
or a pharmaceutically acceptable salt, hydrate or solvate thereof,
with the proviso that Ar₁ and Ar₂ can not be at the same time both phenyl,
for use in the treatment of a disease which can be improved or prevented by an anti-heparanase activity.

In a preferred embodiment, said Ar₁ and Ar₂ are both 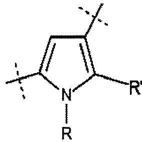 wherein R and R' have the meanings above defined.

In a more preferred embodiment, said compound has the following formula (II): 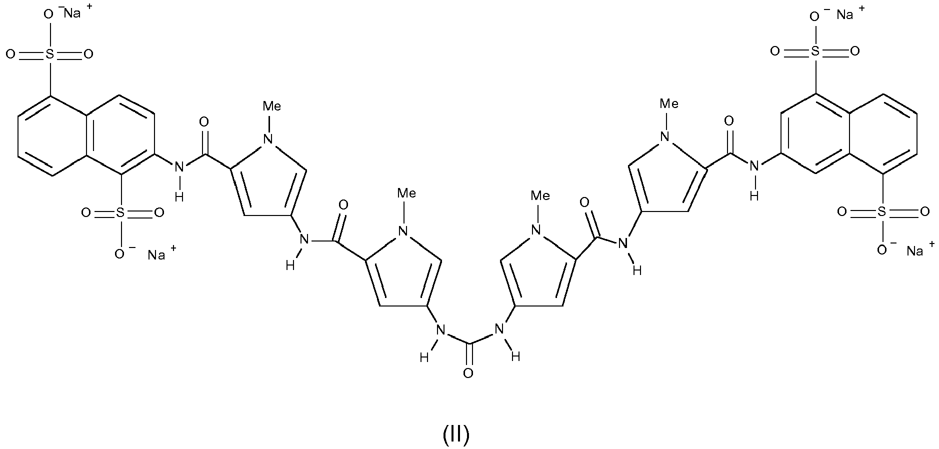 wherein "Me" indicates a methyl group.

In a preferred embodiment, said disease is selected from the group consisting of diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

In another embodiment, said disease is selected from the group consisting of tumor, metastasis and multiple sclerosis. Preferably, for the treatment of one or more of said diseases said compound of formula (I) is not FCE27266 (compound of formula (II)).

It is also an object of the present invention a compound of the above mentioned formula (I) wherein said Ar₁ and Ar₂ are different from each other.

In particular, said compound has the following formula (III): 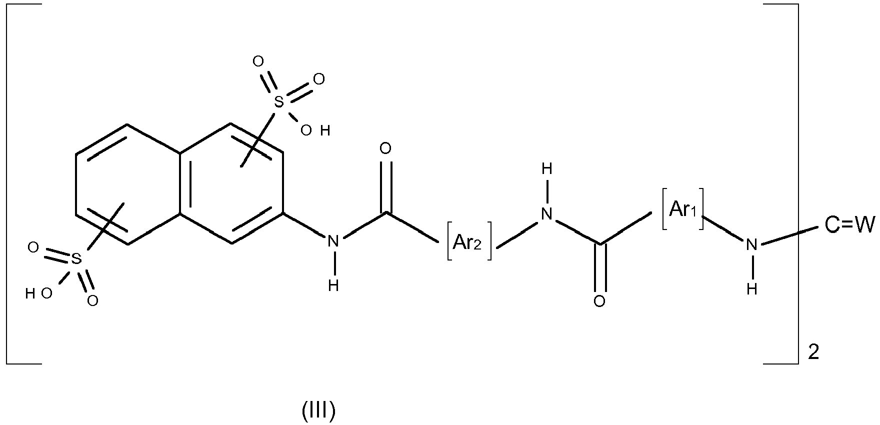 wherein
Ar₁ and Ar₂ are different from each other and selected from 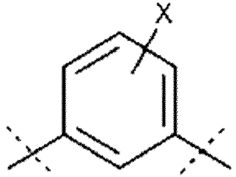
wherein X is selected from the group consisting of H, C₁-C₄alkyl, linear or branched, halogen and C₁-C₄alkyl substituted by one or more halogens,
and 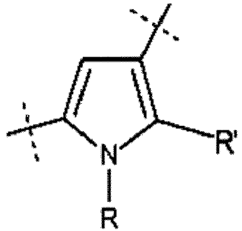
wherein each of R and R' is independently selected from the group consisting of H, C₁-C₄ alkyl, linear or branched, C₁-C₄alkyl substituted by one or more halogens, and cyclopropyl;
and W is selected from the group consisting of O, S and NH,
the enantiomers, diastereoisomers, and mixtures thereof,
or a pharmaceutically acceptable salt, hydrate or solvate thereof.

Indeed, it has been found that said compounds of formula (III) are characterized by a high power of inhibiting heparanase and are therefore useful for the treatment of all pathologies and conditions gaining benefit from the inhibition of the heparanase.

In a preferred embodiment, in the compound of formula (III) W is O.

In a preferred embodiment, in the compound of formula (III), X is H.

In a preferred embodiment, in the compound of formula (III), R is methyl and R' is H.

A process for the preparation of the compound of formula (III), as will be better defined below, is also an object of the present invention.

It is also an object of the invention the compound of formula (III) for use as a medicament.

A further object of the present invention is the a compound of formula (III) for use for the treatment of a disease which can be improved or prevented by an anti-heparanase activity.

In a preferred embodiment, said disease is selected from the group consisting of tumors, primary tumours, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

A further object of the invention is a pharmaceutical composition containing as active ingredient a compound of formula (III) and at least one pharmaceutically acceptable vehicle and/or excipient.

### Detailed description of the invention

### Definitions

Within the meaning of the present invention, the term "alkyl" refers to linear or branched alkyl groups having from 1 to 4 carbon atoms.

Within the meaning of the present invention, the term "anti-heparanase activity" refers to an activity sufficient to inhibit partially or totally the enzyme heparanase.

Within the meaning of the present invention, the term "substituted by" or "substituted with" means that one or more hydrogen atoms of an hydrocarbon are replaced by an atom or a group of atoms (a substituent).

Within the meaning of the present invention, the dotted lines in the above structures indicate a bond with an adjacent group, as evident for a skilled person. For example, the following chemical structure 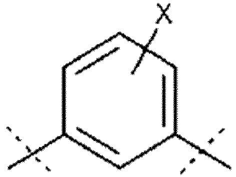 means that the phenyl group is bound with the two adjacent groups in the indicated positions on the ring.

According to the present invention, the compounds of formula (I) are endowed with anti-heparanase activity. Therefore, they can be advantageously used for the treatment of diseases which can be improved or prevented by the inhibition of heparanase activity.

Exemplary diseases which can be treated by compounds of formula (I) are reported above.

In a preferred embodiment of the present invention, in the compound of formula (I) for the use according to the present invention said Ar₁ and Ar₂ are both 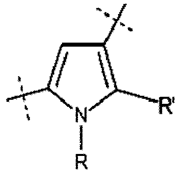 wherein R and R' have the meanings above defined.

Even more preferably, the compound of formula (I) to be used according to the present invention is disodium 2,2'-{carbonylbis[imino(1-methyl-1*H*-pyrrole-4,2-diyl) carbonylimino(1-methyl-1*H*-pyrrole-4,2-diyl)carbonylimino]}dinaphthalene-1,5-disulfonate salt (FCE27266). Indeed, it has been found that such compound is able to efficiently inhibit heparanase activity and therefore it can be used in all diseases and conditions wherein an anti-heparanase activity can be beneficial.

In an embodiment, FCE27266 (compound of formula (II)) is not used for the treatment of cancer, metastasis and/or multiple sclerosis.

Compounds of formula (I) can be obtained according to known procedures. For example, the procedures disclosed in WO9110649 can be followed. Compound of formula (II) (FCE27266) can be obtained, for example, according to the procedures disclosed in WO9420095.

In an exemplary process to obtain the compound of formula (II), a solution of 1-methyl-4-nitro-1*H*-pyrrole-2-carbonyl chloride in 1,4-dioxane is reacted with disodium-2-amino naphthalene-1,5-disulfonate salt and sodium acetate to give 2-(1-Methyl-4-nitro-1H-pyrrole-2-carboxamido)naphthalene-1,5-disulfonic acid disodium salt. Disodium-2-(1-Methyl-4-nitro-1H-pyrrole-2-carboxamido)naphthalene-1,5-disulfonate salt is hydrogenated, for example over Pd/C, to afford disodium 2-{[(4-amino-1-methyl-1*H*-pyrrol-2-yl)carbonyl]amino}naphthalene-1,5-disulfonate salt. Disodium 2-{[(1-methyl-4-{[(1-methyl-4-nitro-1*H*-pyrrol-2-yl)carbonyl]amino}-1*H*-pyrrol-2-yl)carbonyl]amino}naphthalene-1,5-disulfonate salt is then synthesized adding 1-methyl-4-nitro-1*H*-pyrrole-2-carbonyl chloride to the previously obtained compound. Disodium-2-{[(4-{[(4-amino-1-methyl-1*H*-pyrrol-2-yl)carbonyl]amino}-1-methyl-1*H*-pyrrol-2-yl)carbonyl]amino}naphthalene-1,5-disulfonate salt chloride is then obtained by catalitic hydrogenation. Finally, the compound of formula (II) is obtained by adding phosgene in toluene diluited with 1,4-dioxane to a solution of disodium 2-{[(4-{[(4-amino-1-methyl-1*H*-pyrrol-2-yl)carbonyl]amino}-1-methyl-1*H*-pyrrol-2-yl)carbonyl]amino}naphthalene-1,5-disulfonate hydrochloride and sodium acetate.

The use of pharmaceutical compositions containing at least one compound of formula (I) as an active ingredient, in an amount such as to produce a significant therapeutic effect, together with pharmaceutically acceptable vehicle and/or excipients for the treatment of diseases which can be improved or prevented by an anti-heparanase activity is also within the scope of the present invention.

The pharmaceutical composition comprising the compound of formula (I) can also contain one or more further active ingredients, for example chemotherapeutic agents. In particular, when the pharmaceutical composition is used for the treatment of a tumor, it preferably comprises also a chemotherapeutic agent. Indeed in some embodiments, the compounds of formula (I) act by modifying the tumor microenvironment to affect cell growth and spread as well as to facilitate the tumor killing action of other agents. Therefore, the combination of the compound of formula (I) with any chemotherapeutic agent can be particularly useful. The chemotherapeutic agent can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

For example, when the disease to be treated is multiple myeloma (MM), the compounds of formula (I) can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

In an embodiment of the invention the compound of formula (I) is used as an adjuvant of a chemotherapeutic agent or drug, in particular in the treatment of a tumor disease or metastasis. For adjuvant it is intended an agent that modifies and/or facilitate the effect of another agent. In the present case, it is intended that in some embodiments the compound of formula (I) is administered in concomitance with a chemotherapeutic drug to enhance, improve or facilitate the tumor killing action of the chemotherapeutic drug. In this embodiment, the compound of formula (I) does not directly exercise its action on the tumor but, thanks to its inhibitory action on the heparanase, it modifies the tumor microenvironment and facilitates the activity of the chemotherapeutic drug.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

New compounds of the above disclosed formula (III) are also objects of the present invention.

According to the present invention, a C₁-C₄alkyl group can be an alkyl with one, two, three or four carbon atoms. It can be linear or branched. For example, it can be a methyl, ethyl, n-propyl, i-propyl, n-butyl, t-butyl. Preferably, it is methyl.

When an halogen is present, it is selected from the group consisting of: fluorine (F), chlorine (Cl), bromine (Br) and iodine (I). Preferably, it is fluorine (F).

When X or R or R' is a C₁-C₄alkyl substituted with one or more halogen, the halogen can be any of fluorine (F), chlorine (Cl), bromine (Br) and iodine (I), preferably it is fluorine (F). The C₁-C₄alkyl can be substituted, for example, with one, two or three halogens; in a preferred embodiment, it is substituted with three halogens. In a preferred embodiment, it is methyl, preferably substituted by three halogens. In a more preferred embodiment, it is CF₃.

The SO₃H groups can be present on any position of the naphthalene ring. Preferred positions are positions 1 and 5 on the naphthalene ring. In a preferred embodiment, the SO₃H groups are salified, for example with an inorganic base such as sodium hydroxide. Indeed, in a preferred embodiment the compounds of the invention are bis[1,5-Naphthalene-disulfonic acid disodium salt] derivatives.

Any nitrogen group can be protected. The protective group is preferably selected from tert-butoxycarbonyl, benzyloxycarbonyl and 9-fluorenylmethyloxycarbonyl.

In the compound of formula (III) Ar₁ and Ar₂ have different meanings.

When Ar₁ or Ar₂ is

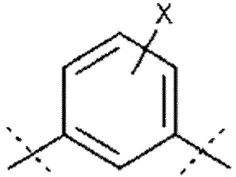

X is preferably selected from H, CH₃, F and CF₃.

In particular, X is preferably CF₃. Indeed, it has been found that fluorine atoms increase interaction with heparanase.

X can be present on any position of the phenyl ring. Preferred positions are 4 and 5.

When Ar₁ or Ar₂ is

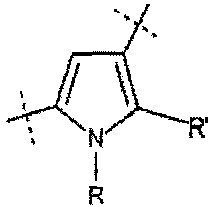

R is preferably selected from CH₃, CF₃ and cyclopropyl and R' is preferably selected from H, CH₃ and CF₃.

In particular, R can advantageously be selected from methyl and cyclopropyl in order to modulate the lipophilicity of the molecule and influence the free rotation of the amide linkages.

R' is preferably H.

In a preferred embodiment of the invention, W is O.

In an embodiment, Ar₁ is 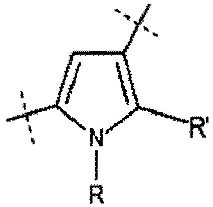 and Ar₂ is 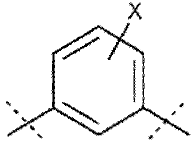 wherein X, R and R' have the meanings above defined.

In an alternative embodiment, Ar₁ is 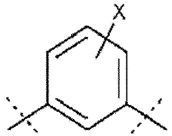 and Ar₂ is 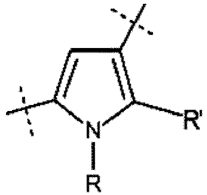 wherein X, R and R' have the meanings above defined.

In a preferred embodiment of the invention, W is O, Ar₁ is 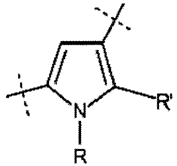 and Ar₂ is 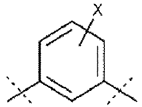 wherein X, R and R' have the meanings above defined. In this embodiment, X is preferably chosen from H and CH₃, R is preferably CH₃ and R' is preferably H.

In another preferred embodiment of the invention, W is O, Ar₁ is 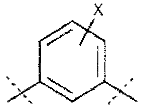 and Ar₂ is 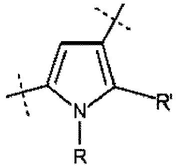 wherein X, R and R' have the meanings above defined. In this embodiment, X is preferably chosen from H, CF₃ and F, R is preferably CH₃ and R' is preferably H.

In a preferred embodiment of the invention, W is O, Ar₁ is 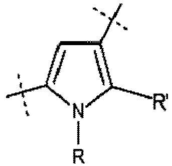 and Ar₂ is 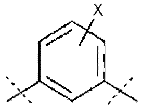 wherein X, R and R' have the meanings above defined. In this embodiment, X is preferably chosen from H and CH₃, R is preferably CH₃ and R' is preferably H.

In another preferred embodiment of the invention, W is O, Ar₁ is 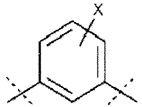 and Ar₂ is 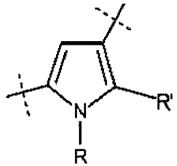 wherein X, R and R' have the meanings above defined. In this embodiment, X is preferably chosen from H, CF₃ and F, R is preferably CH₃ and R' is preferably H.

In another embodiment of the invention, W is S, Ar₁ is 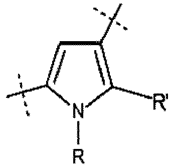 and Ar₂ is 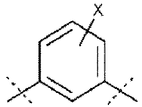 wherein X, R and R' have the meanings above defined.

In another embodiment of the invention, W is S, Ar₁ is 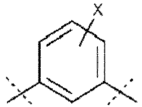 and Ar₂ is 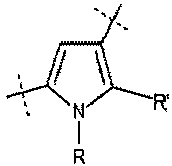 wherein X, R and R' have the meanings above defined.

In another embodiment of the invention, W is NH, Ar₁ is 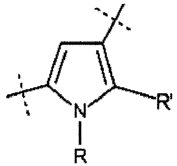 and Ar₂ is 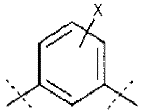 wherein X, R and R' have the meanings above defined.

In another embodiment of the invention, W is NH, Ar₁ is 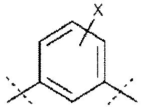 and Ar₂ is 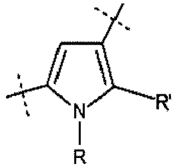 wherein X, R and R' have the meanings above defined.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (III) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (III) and inorganic bases, such as sodium, potassium, calcium and aluminum hydroxides, as well as those with organic bases such as, for example, lysine, arginine, N-methyl-glucamine, triethylaminetriethanolamine, dibenzilamine, methylbenzilamine, d-(2-ethyl-hexyl)-amine, piperidine, N-ethylpiperidine, N,N-di-ethylaminoethylamine, N-ethylmorpholine, 3-phenethylamine, N-benzil-3-phenethylamine, N-benzil-N,N-dimethylamine. A preferred salt is sodium salt.

In a preferred embodiment of the invention, the compound of formula (III) is in the form of a sodium salt.

Also, pharmaceutically acceptable hydrates or solvates of the compound of formula (III) are included in the scope of the present invention. In particular, they can be any hydrate or solvate commonly used in the art.

Pharmaceutically acceptable salts and their preparation are within the common knowledge of the skilled person and general reference is made to the relevant literature, such as, for example Pharmaceutical Salts and Co-crystals, Johan Wouters, Luc Quéré, Royal Society of Chemistry, 2011.

Preferred compounds according to the present invention are reported in the following Table 1:

**Table 1. Compounds of the invention.**

| N. | Internal ID | Formula and Chemical name |
|---|---|---|
| 37a | SST0546NA1 | 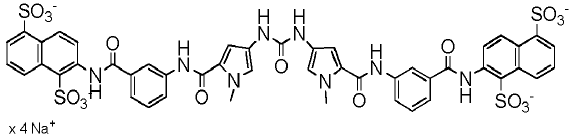 |
| | | Disodium 2,2'-{carbonylbis[imino(1-methyl-1*H*-pyrrole-4,2-diyl)carbonylimino-3,1-phenylenecarbonylimino]}dinaphthalene-1,5-disulfonate salt |
| 31a | SST0548NA1 | 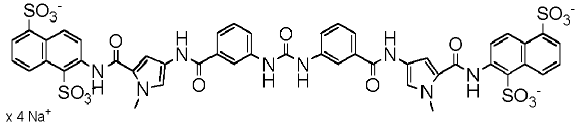 |
| | | Disodium 2,2'-{carbonylbis[imino-3,1-phenylenecarbonylimino(1-methyl-1*H*-pyrrole-4,2-diyl)carbonylimino]}dinaphthalene-1,5-disulfonate salt |
| 37b | SST0562NA1 | 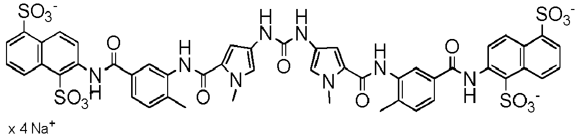 |
| | | Disodium 2,2'-{carbonylbis[imino(1-methyl-1*H*-pyrrole-4,2-diyl)carbonylimino(4-methyl-3,1-phenylene)carbonylimino]}dinaphthalene-1,5-disulfonate salt |
| 31b | SST0591NA1 | 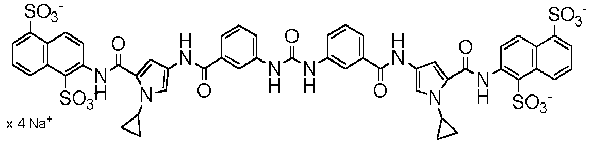 |
| | | Disodium 2,2'-{carbonylbis[imino-3,1-phenylenecarbonylimino(1-cyclopropyl-1*H*-pyrrole-4,2-diyl)carbonylimino]}dinaphthalene-1,5-disulfonate salt |
| 31c | SST0592NA1 | 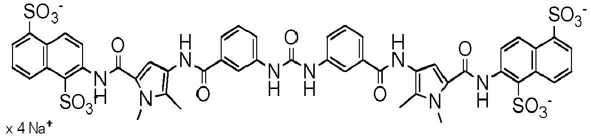 |
| | | Disodium 2,2'-{carbonylbis[imino-3,1-phenylenecarbonylimino(1,5-dimethyl-1*H*-pyrrole-4,2-diyl)carbonylimino]}dinaphthalene-1,5-disulfonate salt |
| 31e | SST0612NA1 | 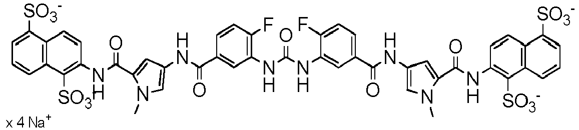 |
| | | Disodium 2,2'-{carbonylbis[imino(4-fluoro-3,1-phenylene)carbonylimino(1-methyl-1*H*-pyrrole-4,2-diyl)carbonylimino]}dinaphthalene-1,5-disulfonate salt |
| 31d | SST0613NA1 | 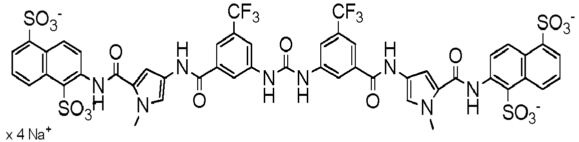 |
| | | Disodium 2,2'-{carbonylbis[imino[5-(trifluoromethyl)-3,1-phenylene]carbonylimino(1-methyl-1*H*-pyrrole-4,2-diyl)carbonylimino]}dinaphthalene-1,5-disulfonate salt |

A process for the preparation of the compound of formula (III) is also within the scope of the present invention.

The compounds of the present invention can be synthesized by many methods available to those skilled in the art of organic chemistry. General synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared by methods described in the general schemes are given in the intermediates and examples section set out hereinafter.

The compounds of the present invention can be prepared in a number of ways known to one skilled in the art of organic synthesis. The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed. This will sometimes require a modification of the order of the synthetic steps or the selection of one particular process scheme over another in order to obtain a desired compound of the invention. All such abilities are well within the knowledge and abilities of the skilled person.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006)).

Also, the skilled in the art can easily alter the reagents and reaction conditions exemplified in the schemes below to include any combination of substituents encompassed by the present invention. Also, the skilled artisan can easily use interchangeable steps for each synthetic process and incorporate isolation and/or purification steps as deemed necessary.

All the conditions and reagents not explicitly mentioned below can be easily chosen by the skilled in the art according to the general knowledge in the field.

Starting materials useful for preparing the compounds of the invention and intermediates therefor are commercially available or can be prepared by well known synthetic methods.

### Exemplary synthesis processes

It is herein described an exemplary process for obtainment of compounds of formula (III) wherein W is O, Ar₁ is 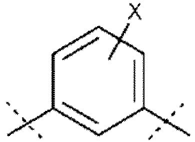 and Ar₂ is 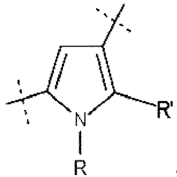 wherein X, R and R' have the meanings above defined.

With reference to the following Scheme 2, the amino derivative 4 can be obtained by acylation of the commercially available 2-amino naphthalene-1,5-disulfonic acid disodium salt 1 with the appropriate pyrrole moiety 2 affording the intermediate 3 (step i), which is easily reduced to the amine compound 4 by catalytic hydrogenation (step ii), for example using as a catalyst palladium on carbon (Pd/C). The condensation with the appropriate acid chloride derivative 5 gives the nitro compound 6 (step iii).The latter derivative can be reduced using catalytic hydrogenation affording compound 7 (step iv) for example using as a catalyst palladium on carbon (Pd/C). The final condensation reaction (step v) to obtain a compound according to the invention 8 is carried out by means of a suitable condensation agent, for example the phosgene.

In each step, appropriate solvents and reagents can be used, for example dioxane, toluene or sodium acetate.

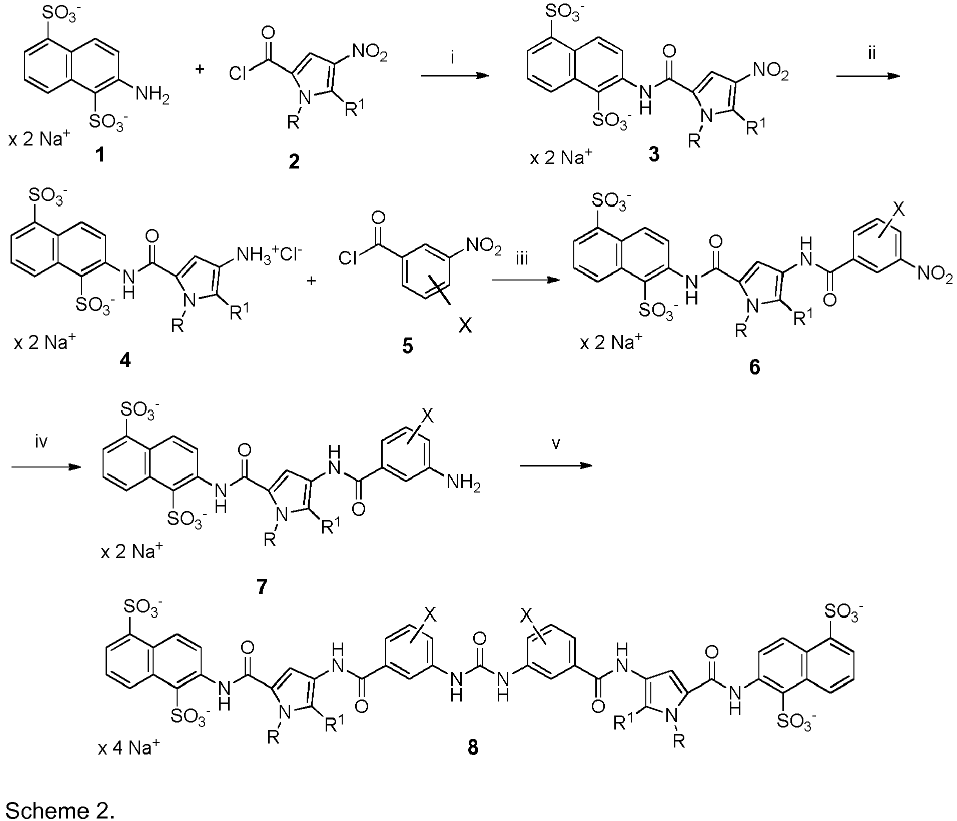

It will be appreciated by the skilled person that this process can be equally used to obtain compounds wherein the SO₃H groups are located on different positions of the naphthalene ring by choosing the suitable starting compound.

Compounds having the meanings of X, R and R' according to the present invention can be obtained through this exemplary process by choosing the appropriate starting compound, in particular by choosing the appropriate pyrrole moiety (2) and the appropriate acid chloride derivative (5). All such compounds are commercially available or they can be obtained by suitable and simple modifications of known compounds according to the general knowledge of the skilled person in the field.

For example, commercially available 1-methyl-4-nitro-1H-pyrrole-2-carbonyl chloride, 1-cyclopropyl-4-nitro-1h-pyrrole-2-carbonyl chloride or 1,5-dimethyl-4-nitro-1h-pyrrole-2-carbonyl chloride can be used as the pyrrole moiety (compound 2) in step (i) above to obtain the corresponding final compounds.

Commercially available compounds such as 3-nitro-benzoyl-chloride, 3-nitro-5-(trifluoromethyl)benzoyl chloride and 4-fluoro-3-nitro-benzoyl chloride can be used as the acid chloride derivative (compound 5) in step (iii) above to obtain the corresponding final compounds.

Compounds wherein Ar₁ and Ar₂ have the meanings above defined and wherein W is S can be obtained by the same procedure above described and shown in Scheme 2 with appropriate modifications, in particular by choosing the suitable condensation agent in step (v), for example thiocarbonyldiimidazole.

Compounds wherein Ar₁ and Ar₂ have the meanings above defined and wherein W is NH can be obtained by the same procedure above described and shown in Scheme 2 with appropriate modifications, in particular by choosing the suitable condensation agent in step (v), for example di(imidazole-1-yl)methanimine.

It is herein described an exemplary process for obtainment of compounds wherein W is O, Ar₁ is 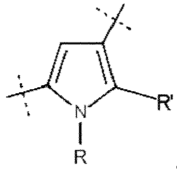 and Ar₂ is 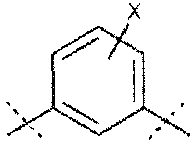 wherein X, R and R' have the meanings above defined.

With reference to the following Scheme 3, the condensation of commercially available 2-amino naphthalene-1,5-disulfonic acid disodium salt 9 with a 3-nitro-benzoyl-chloride 10, optionally substituted with X, wherein X have the meanings above defined, gives the nitro derivative 11 (step i) which can be easily reduced by catalytic hydrogenation to amine 12 (step ii), for example using as a catalyst palladium on carbon (Pd/C). The latter amine can be reacted with the appropriate pyrrole moiety 13, for example in the form of a chloride, to give the nitro derivative 14 (step iii). The final condensation reaction (step v) to obtain the urea derivatives 16 is possible by means of a suitable condensation agent, for example the phosgene, using the amine 15 which can be obtained from derivative 14 by catalytic hydrogenation (step iv), for example using as a catalyst palladium on carbon (Pd/C).

In each step, appropriate solvents and reagents can be used, for example dioxane, toluene or sodium acetate.

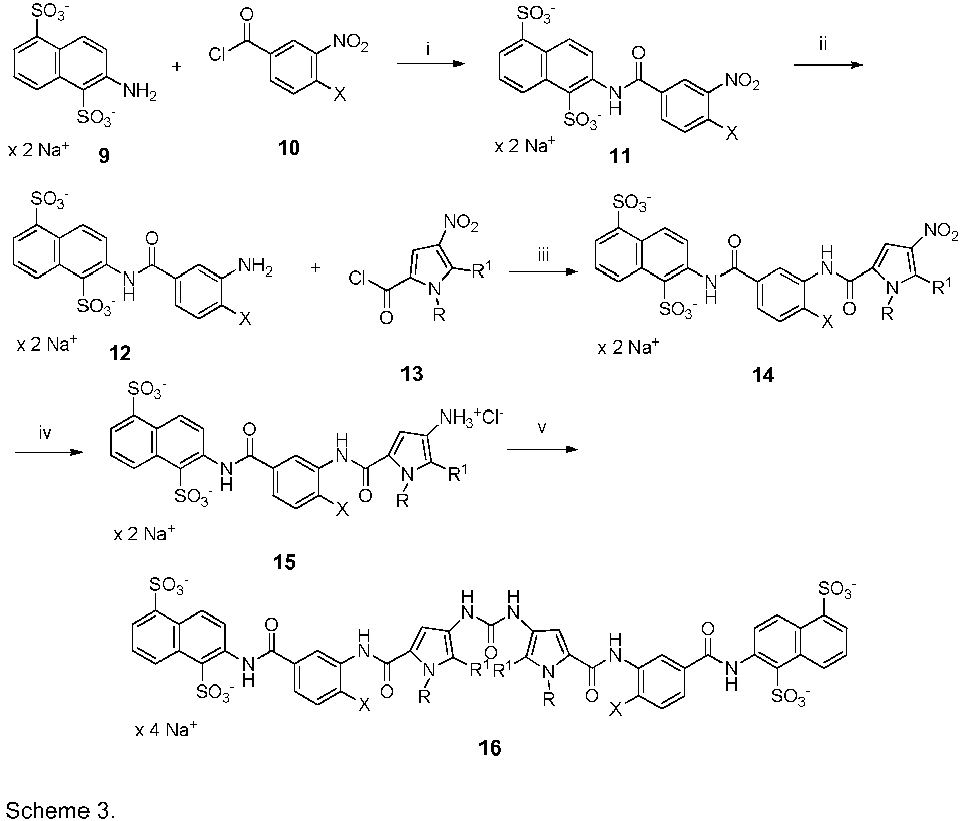

It will be appreciated by the skilled person that this process can be equally used to obtain compounds wherein the SO₃H groups and the X group are located on different positions of the naphthalene and the phenyl ring, respectively, by choosing the suitable starting compounds.

Compounds having the meanings of X, R and R' according to the present invention can be obtained through this exemplary process by choosing the appropriate starting compound, in particular by choosing the appropriate pyrrole moiety (13) and the appropriate phenyl moiety (10). All such compounds are commercially available or they can be obtained by suitable and simple modifications of known compounds according to the general knowledge of the skilled person in the field.

For example, 3-nitrobenzoyl chloride or 4-methyl-3-nitrobenzoyl chloride can be used as a phenyl moiety (compound 10) in step (i) above to obtain the corresponding final compounds.

Compounds such as 1-methyl-4-nitro-pyrrole-2-carbonyl chloride can be used as a pyrrole moiety (compound 13) in step (iii) above to obtain the corresponding final compounds. Compounds wherein Ar₁ and Ar₂ have the meanings above defined and wherein W is S can be obtained by the same procedure above described and shown in scheme 3 with appropriate modifications, in particular by choosing the suitable condensation agent in step (v), for example thiocarbonyldiimidazole.

Compounds wherein Ar₁ and Ar₂ have the meanings above defined and wherein W is NH can be obtained by the same procedure above described and shown in scheme 3 with appropriate modifications, in particular by choosing the suitable condensation agent in step (v), for example di(imidazole-1-yl)methanimine.

All the transformations above described are only exemplary synthetic procedures already known in organic chemistry and well known to the experts in the field.

Any variation or combination of substituents of compounds of formula (III) which is within the scope of the present invention and is not explicitly shown or described in the above processes, can be easily obtained starting from the exemplary processes described above with suitable modifications, for example in the starting compounds or reagents, that are well within the knowledge of the skilled person in the field of organic chemistry. Reference can be made, for example, to the handbook "March's Advanced Organic Chemistry, Reactions, Mechanism and structures.", Michael B. Smith, Jerry March, last edition.

### Medical uses

It has been found that compounds of formula (I) and (III) are endowed with high inhibitory activity of heparanase. Therefore, they can be advantageously used in the treatment of any disease or condition which can be ameliorated or prevented by an anti-heparanase activity.

The heparanase has a critical role in modulating the microenvironment via the ECM (extracellular matrix) remodeling and thus influencing a multitude of both normal and disease-related processes, as inflammation, blood coagulation, wound healing, angiogenesis, fibrosis and tumor cell growth, invasion and metastasis (for a review of the roles and activities of heparanase see for example "Heparanase: From basic research to therapeutic applications in cancer and inflammation", Vlodavsky I., Singh P., Boyango I., Gutter-Kapon L., Elkin M., Sanderson RD, Ilan N., Drug Resist Updat. 2016 Nov;29:54-75). Heparanase has also been recently reported to play a fundamental role in many other pathologies, such as nephropathies of different origin, severe infective disorders, gastrointestinal diseases, osteolysis in bone metastases as well as in other bone tissue related pathologies, atherosclerosis, cardiovascular disorders and cutaneous aging. The panorama of diseases affected by heparanase expression and activity also includes the so-called rare diseases, such as amyloidosis, hereditary multiple exostoses and idiopathic avascular necrosis of bone as well as pathologies that are not rare in terms of incidence, but are rarely diagnosed, such as vulvodynia (Rivara et al., 2016).

Therefore, the compounds of formula (III) can be advantageously used in a broad range of diseases.

In particular, heparanase expression is enhanced in cancers, including various carcinomas, sarcomas and hematological malignancies, and it has been demonstrated that it correlates with increased tumor size, tumor angiogenesis, enhanced metastasis and poor prognosis. Indeed, treatments of tumor-bearing mice with heparanase-inhibiting compounds has been shown to markedly attenuate tumor progression further supporting an anti-heparanase therapy for multiple types of cancer (Vlodavski et al., 2016).

Therefore, the compounds of the invention, thanks to their demonstrated activity as heparanase inhibitors, can be advantageously used in the treatment of tumors and metastasis.

In particular, they can be used in the treatment of any kind of tumor, both solid and liquid. For example they can be used in the treatment of solid tumors, such as for example glioma and sarcomas, such as fibrosarcoma and osteosarcoma, and in the treatment of hematological malignancies.

The involvement of heparanase in inflammatory reactions has also been shown (Vlodavski et al., 2016) thus supporting the use of inhibitors of heparanase also in diseases characterized by or related with acute and chronic inflammation, such as colitis, atherosclerosis, pancreatitis, Chron's disease, psoriasis and others.

More recently, heparanase overexpression has been documented to be related to the endothelial mesenchymal transition (EMT; Masola V et al. Specific heparanase inhibition reverses glucose-induced mesothelial-to-mesenchymal transition Nephrol. Dial. Transplant 2017; 1-9) and thus heparanase inhibition may be also beneficial in those pathologic processes involving EMT, as fibrosis occurring at different organs.

Examples of diseases which can be treated by compounds of formula (III) are tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

The skilled in the art, for example a physician, can identify and recognize if a disease can be ameliorated by an anti-heparanase activity on the basis of the common knowledge in the field. For example, for a broad, even though not exhaustive, overview, on possible clinical applications of heparanase inhibitors, reference can be made to the reviews of Rivara et al., 2016 and Vlodavski et al., 2016.

Also, tests are known in the field and available to the skilled person to evaluate if a compound is endowed with an anti-heparanase activity, for example the AGAIA assay (Hammond E. et al. Anal. Biochem. 2010, 396, 112).

Furthermore, it has been found that compounds of formula (III) are able to inhibit invasion of tumor cells. This renders them particularly suitable for the use in the treatment of tumors, in particular for the inhibition of tumor growth.

Compounds of the invention are particularly advantageous also for non-tumor applications since they are endowed with a strong anti-heparanase activity and therefore they can already be efficiently used at very low doses at which they are not cytotoxic. Indeed, in some embodiments they are selective heparanase inhibitors already at concentrations in which they do not interfere significantly with cell proliferation. This renders them particularly useful for therapeutic applications wherein a cytotoxic effect is undesired. Also, it has been found that such compounds at not cytotoxic concentrations are already able to inhibit invasion of tumor cells thus being particularly useful in the treatment of tumors and metastasis, also as adjuvants of chemotherapeutic agents. The compounds of the invention are also able to interfere with adhesion properties of cancer cells and they have an inhibitory effect against tumor transcription genes encoding for proangiogenic factors thus further supporting their therapeutic use in cancer diseases and metastasis.

The skilled person in the field knows how to determine the suitable dose in view of the desired application and his general knowledge in the medical and pharmaceutical field and by carrying out routine experimentation.

Another object of the present invention is a pharmaceutical composition containing at least one compound of formula (III) as an active ingredient, in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical compositions according to the invention comprising the compound of formula (III) can also contain one or more further active ingredients, for example chemotherapeutic agents. In particular, when the pharmaceutical composition is used for the treatment of a tumor, it preferably comprises also a chemotherapeutic agent. Indeed in some embodiments, the anti-heparanase compounds of the invention act by modifying the tumor microenvironment to affect cell growth and spread as well as to facilitate the tumor killing action of other agents. Therefore, the combination of the compounds of the invention with any chemotherapeutic agent can be particularly useful. The chemotherapeutic agent can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

For example, when the disease to be treated is multiple myeloma (MM), the compounds of the invention can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (III) with suitable excipients, stabilizers and/or pharmaceutically acceptable diluents.

It is also an object of the invention the use of such pharmaceutical composition comprising a compound of formula (III) as a medicament, in particular in the treatment of a disease which can be improved or prevented by an anti-heparanase activity. More in particular, the above mentioned diseases can be treated by such pharmaceutical composition.

In an embodiment, the compounds of the invention can also be used for cosmetic applications thanks to their activity of inhibition of heparanase. Indeed, heparanase is produced in the skin by epidermal keratinocytes composing the epidermis and fibroblasts or vascular endothelial cells of the dermis and it decomposes heparan sulfate proteoglycan, which regulates differentiation and growth of the epidermis. It is known that heparanase can promote the formation of wrinkles; indeed, inhibition of heparanase activity suppresses the release of growth factors that accompanies decomposition of heparan sulfate, and allows migration of growth factors between the epidermis and dermis to be controlled, thereby aiding in anti-aging and anti-wrinkles effects of the skin (see for example EP2484349, US2014080878 and the work "Hyperpigmentation in human solar lentigo is promoted by heparanase-induced loss of heparan sulfate chains at the dermal-epidermal junction." Iriyama S, Ono T, Aoki H, Amano S. Dermatol Sci. 2011 Dec;64(3):223-8).

In view of the above and of the heparanase inhibition activity of the compounds of the invention, it is also an object of the present invention the cosmetic use of the compounds of formula (I) or (III) for prevention or treatment of skin aging. In particular, they can be used for preventing or suppressing the formation of wrinkles, in particular wrinkles caused by reduced level of heparanase sulfate in the epidermal basal membrane.

The compounds of the invention are particularly suitable for such cosmetic applications due to their low cytotoxicity. In particular, due to their potent anti-heparanase activity, they can already exert their effect at very low doses, at which they are not cytotoxic and thus suitable for cosmetic uses.

It is also an object of the invention, a cosmetic composition comprising the compound of formula (III) and cosmetically acceptable excipients and/or vehicles. Such excipients or vehicles can be chosen by the skilled person among the cosmetic ingredients commonly used in the field. In particular, they are components commonly used in external preparations. Such cosmetic composition can be used in the prevention or suppression of wrinkles or in any other anti-aging applications. It is preferably applied and administered topically.

The following examples further illustrate the invention.

### EXAMPLES

### General Remarks:

¹H and ¹³C NMR data were obtained with a Varian VXR 200 spectrometer and a Varian Mercury Plus 400 spectrometer, respectively. Peak positions are given in parts per million (*δ*) downfield, and *J* values are given in hertz. Positive-ion electrospray ionization (ESI) mass spectra were recorded on a double-focusing Finnigan MAT 95 instrument with BE geometry. TLC (Thin Layer Chromatography) was carried out using glass plates coated with silica gel 60 F₂₅₄ by Merck, and compounds were visualized by UV detection or with aqueous KMnO₄. Flash column chromatography was performed using 230-400 mesh silica gel and the indicated solvent system. Organic solutions were dried over anhydrous Na₂SO₄. Solvents and reagents that are commercially available were purchased from Aldrich (Sigma-Aldrich) or Alfa Aesar (Johnson Matthey Company) and were used without further purification unless otherwise noted.

### EXAMPLES 1-5

The compounds **31 b,c** where the first pyrrole heterocyclic ring was substituted by 1-cyclopropyl-pyrrole or 1,5-dimetil-pyrrole residues and **31 a,d,e** where the second pyrrole heterocyclic ring was substituted by benzoic acid or 5-trifluoromethyl-benzoic acid or 4-fluorobenzoic acid were synthesized following procedures described in the following Scheme 4. The amino derivatives **4,** 27 **a,b** were obtained by acylation of the commercially available 2-amino naphthalene-1,5-disulfonic acid disodium salt **1** with the appropriate pyrrole moiety **2, 25 a,b** affording in good yield the intermediate **26 a,b,c,** which were easily reduced to the amine compounds **4, 27 a,b** by catalytic hydrogenation, for example using a catalyst palladium on carbon (Pd/C). The condensation with the appropriate acid chloride derivatives **28 a,b,c** give the nitro compounds **29 a-e** in a yield between the 70 and 90%. The latter derivatives were reduced using catalytic hydrogenation in quantitative yield affording **30 a-e.** The final condensation reaction to obtain the urea derivatives **31 a-e** was possible by means of the phosgene as condensation agent in a yield between 10 of 20%.

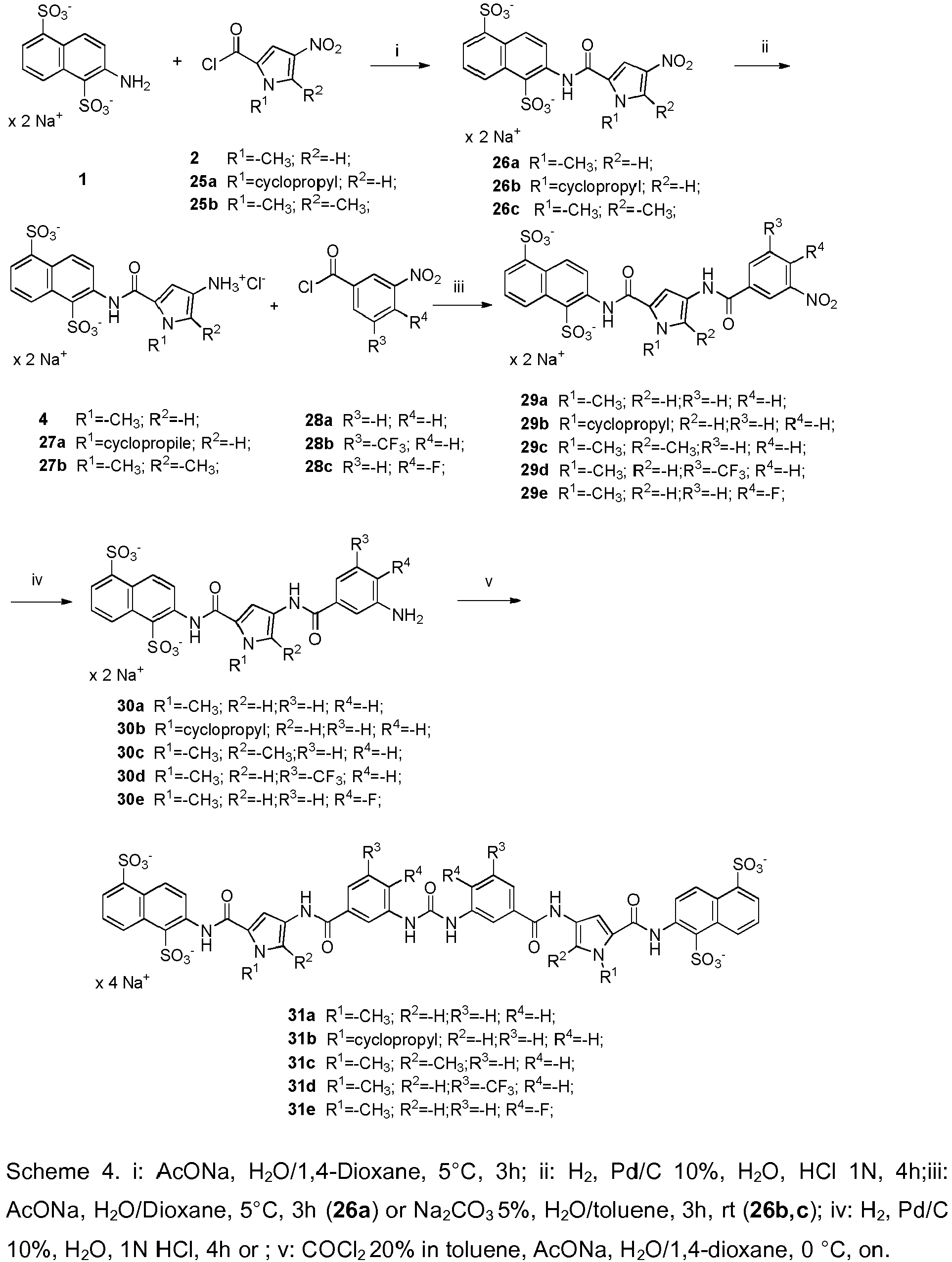

### Example 1

### Disodium 2,2'-{carbonylbis[imino-3,1-phenylenecarbonylimino(1-methyl-1H-pyrrole-4,2-diyl)carbonylimino]}dinaphthalene-1,5-disulfonate salt (SST0548NA1) (31a)

Compound **31a** was synthesized following the procedures described in the following Scheme 5:

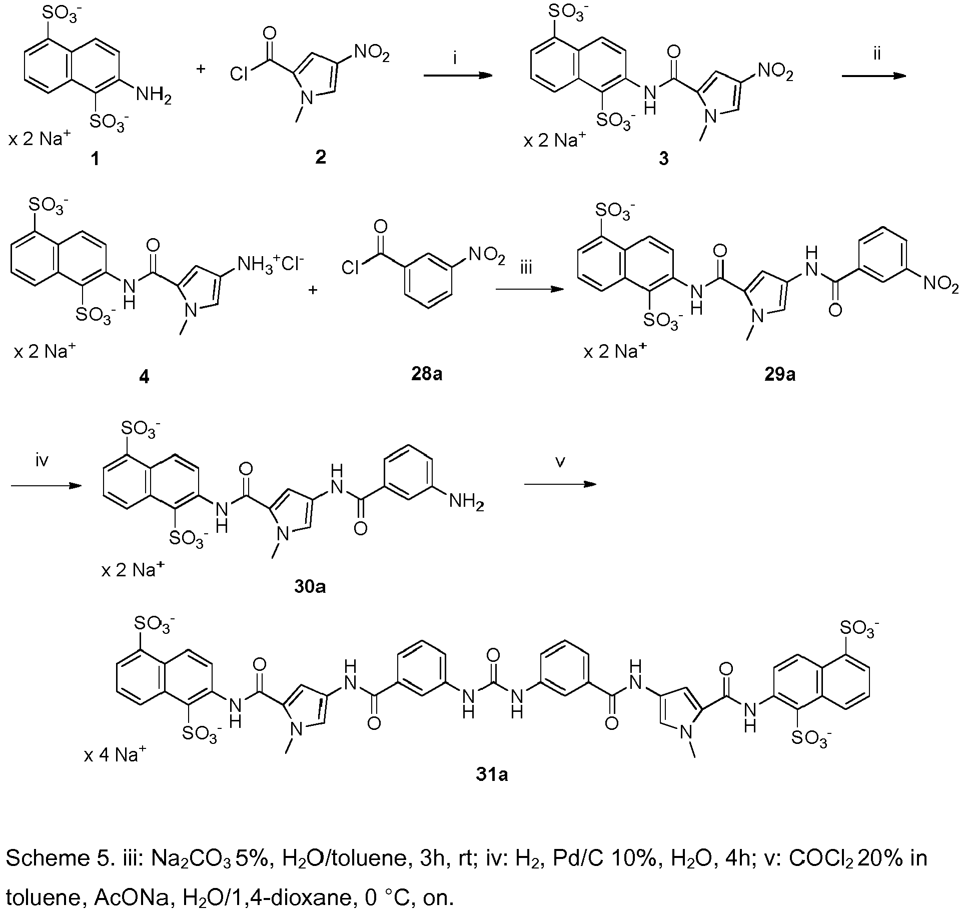

### Step i: 2-(1-Methyl-4-nitro-1H-pyrrole-2-carboxamido)naphthalene-1,5-disulfonic acid disodium salt (3)

A solution of 1-methyl-4-nitro-1*H*-pyrrole-2-carbonyl chloride (950 mg, 5 mmol) in 1,4-dioxane (45 mL) was added dropwise (1 h) to a cold solution (5 °C) of disodium-2-amino naphthalene-1,5-disulfonate salt (1.03 g, 2.96 mmol) and sodium acetate (490 mg, 5.93 mmol) in water (45 mL). After stirring for 3 h at 5 °C, the solution was acidified to pH 4 with HCl 1 N and evaporated to dryness. The residue was treated with ethyl acetate (300 mL), stirred for 4 h and filtered to give 2-(1-Methyl-4-nitro-1 H-pyrrole-2-carboxamido)naphthalene-1,5-disulfonic acid disodium salt 1.34 g (91%), which was used in the next step without further purification.

¹H-NMR 400 MHz (DMSO-*d*₆) δ: 4.03 (s, 3H), 7.38-7.40 (m, 2H), 7.86 (d, 1H, *J*=6,8 Hz), 8.29 (d, 1H, *J*=1.2 Hz), 8.65 (d, 1H, *J*=9.6 Hz), 8.87 (d, 1H, *J*=9.6 Hz), 9.00 (d, 1H, *J*=8.8 Hz), 13.00 (s, 1H).

### Step ii: disodium 2-{[(4-amino-1-methyl-1H-pyrrol-2-yl)carbonyl]amino}naphthalene-1,5-disulfonate salt chloride (4)

To a solution of disodium-2-(1-Methyl-4-nitro-1H-pyrrole-2-carboxamido)naphthalene-1,5-disulfonate salt (600 mg, 1.2 mmol) in water (40 mL) was added 1 N HCl (1.2 mmol, 1.2 mL) (pH= 3) and was hydrogenated over 10% Pd/C (catalytic amount) for 4h. The catalyst was filtered off and the resulting solution was concentrated to afford disodium 2-{[(4-amino-1-methyl-1*H*-pyrrol-2-yl)carbonyl]amino}naphthalene-1,5-disulfonate salt chloride 600 mg (98.8%).

¹H-NMR 400 MHz (DMSO-*d*₆) δ: 3.94 (s, 3H), 6.89 (d, 1 H, *J*=2.0 Hz), 7.19 (d, 1H), 7.33-7.37 (m, 1H), 7.80 (d, 1H, *J*=6.0 Hz), 8.66 (d, 1H, *J*=9.6 Hz), 8.82 (d, 1H, *J*=10.0 Hz), 8.97(d, 1H, *J*=9.2 Hz), 9.95 (s, 3H), 12.79 (s, 1H).

### Step iii: disodium 2-[({1-methyl-4-[(3-nitrobenzoyl)amino]-1H-pyrrol-2-yl}carbonyl)amino]naphthalene-1,5-disulfonate salt (29a)

A solution of 3-nitro-benzoyl-chloride (1.04 g, 5.62 mmol) in 45 mL of toluene was added slowly in a solution of 2-{[(4-amino-1-methyl-1*H*-pyrrol-2-yl)carbonyl]amino}naphthalene-1,5-disulfonate salt chloride(1420 mg, 2.8 mmol) in 45 mL of H₂O; a 5% solution of Na₂CO₃ in water was added to the mixture of reaction until pH 4. After stirring for 3 h at room temperature, the aqueous phase was separated from toluene, was acidified to pH 4 with HCl 1N and was extracted with Et₂O for three times. The acid aqueous phase was isolated and neutralized with a 5% solution of Na₂CO₃ in water (pH 7) and evaporated to dryness to give disodium 2-[({1-methyl-4-[(3-nitrobenzoyl)amino]-1*H*-pyrrol-2-yl}carbonyl)amino]naphthalene-1,5-disulfonate salt 1.7 g (98%).

¹H-NMR 200 MHz (DMSO-*d*₆) δ: 3.95 (s, 3H), 7.08 (d, 1H, *J*=1.6 Hz), 7.36-7.40 (m, 1H), 7.60 (d, 1H, *J*=1.6 Hz), 7.80-7.88 (m, 2H), 8.44-8.47 (m, 2H), 8.70 (d, 1H, *J*=9.8 Hz), 8.82-8.86 (m, 2H), 9.01 (d, 1H, *J*=9.2 Hz).

### Step iv: disodium 2-[({4-[(3-aminobenzoyl)amino]-1-methyl-1H-pyrrol-2-yl}carbonyl)amino]naphthalene-1,5-disulfonate salt (30a)

A solution of disodium 2-[({1-methyl-4-[(3-nitrobenzoyl)amino]-1*H*-pyrrol-2-yl}carbonyl)amino]naphthalene-1,5-disulfonate salt (600 mg, 0.97 mmol) in water (40 mL) was hydrogenated over 10% palladium on carbon (catalytic amount) for 4h. The catalyst was filtered off and the resulting solution was concentrated to afford disodium 2-[({4-[(3-aminobenzoyl)amino]-1-methyl-1*H*-pyrrol-2-yl}carbonyl)amino]naphthalene-1,5-disulfonate salt 560 mg (98%).

¹H-NMR 400 MHz (DMSO-*d*₆) δ: 3.92 (s, 3H), 5.27 (s, 2H), 6.71 (m, 1H), 7.02 (d, 1H, *J*=1.6 Hz), 7.10-7.13 (m, 3H), 7.39-7.34 (m, 1H), 7.53 (d, 1H, *J*=1.6 Hz), 7.81-7.83 (m, 1H), 8.69 (d, 1H, *J*=9.2 Hz), 8.83 (d, 1H, *J*=9.2 Hz), 9.01 (d, 1H, *J*=8.4 Hz).

### Step v: disodium 2,2'-{carbonylbis[imino-3,1-phenylenecarbonylimino(1-methyl-1H-pyrrole-4,2-diyl)carbonylimino]}dinaphthalene-1,5-disulfonate salt (SST0548NA1) (31a)

A 20% solution of phosgene in toluene (0.45 mL, 0.8 mmol), diluited with 1,4-dioxane (1.5 mL), was added slowly to a cold (5°C) solution of disodium 2-[({4-[(3-aminobenzoyl)amino]-1-methyl-1*H*-pyrrol-2-yl}carbonyl)amino]naphthalene-1,5-disulfonate salt (230 mg, 0.4 mmol) and sodium acetate (130 mg, 1.6 mmol) in water (15 mL) and 1,4-dioxane (4 mL). After stirring for 2 h at 5 °C, solvents were evaporated under vacuum and the residue was taken up in methanol. Precipitated salts were filtered off, the filtrate was evaporated and the residue was chromatographed on a silica gel column with CH₂Cl₂:MeOH:H₂O (60:40:4 v/v) as eluent to give disodium 2,2'-{carbonylbis[imino-3,1-phenylenecarbonylimino(1-methyl-1*H-*pyrrole-4,2-diyl)carbonylimino]}dinaphthalene-1,5-disulfonate salt SST0548NA1 48 mg (10%).

¹H-NMR 400 MHz (DMSO-*d*₆) δ: 3.95(s, 3H), 7.05 (d, 1H, *J*=2.0 Hz), 7.34-7.43 (m, 2H), 7.63-7.59 (m, 2H), 7.82-7.93 (m, 3H), 8.70 (d, 1H, *J*=10.0 Hz), 8.83 (d, 1H, *J*=9.6 Hz), 9.01 (d, 1H, *J*=8.4 Hz), 9.09 (s, 1H), 10.52 (s, 1H), 12.69 (s, 1H).

### Example 2

### Disodium 2,2'-{carbonylbis[imino-3,1-phenylenecarbonylimino(1-cyclopropyl-1H-pyrrole-4,2 diyl)carbonylimino]}dinaphthalene-1,5-disulfonate salt (SST0591NA1) (31b)

Compound **31b** was synthesized as described in example 1 using a different pyrrolic derivate in step i (1-cyclopropyl-4-nitro-1*H*-pyrrole-2-carbonyl chloride) and a different procedure in step iii (Scheme 6).

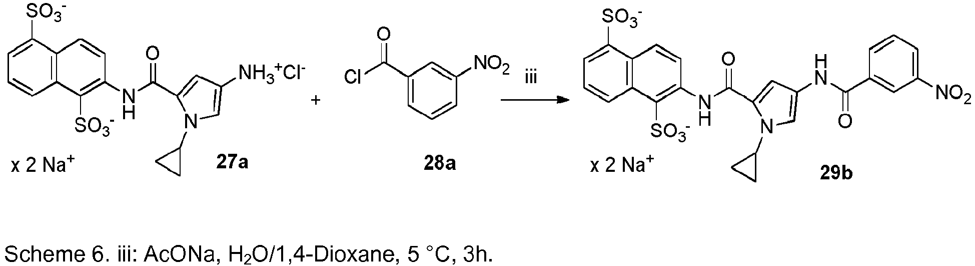

### Step i: disodium 2-{[(1-cyclopropyl-4-nitro-1H-pyrrol-2-yl)carbonyl]amino}naphthalene-1,5-disulfonate salt (26b)

¹H-NMR 200 MHz (DMSO-*d*₆) δ: 1.01-1.11 (m, 4H), 4.17 (m, 1H), 7.33-7.37 (m, 2H), 7.81-7.84 (m, 1H), 8.17 (d, 1H, *J*=1.6 Hz), 8.66 (d, 1H, *J*=9.6 Hz), 8.84 (m, 1H), 8.89 (d, 1H, *J*=8.4 Hz), 12.95 (s, 1H). Yield 80%

### Step ii: disodium 2-{[(4-amino-1-cyclopropyl-1H-pyrrol-2-yl)carbonyl]amino}naphthalene-1,5-disulfonate chloride salt (27a)

¹H-NMR 200 MHz (DMSO-*d*₆) δ: 0.93-1.03 (m, 4H), 3.98 (m, 1H), 6.88 (d, 1H, *J*=1.6 Hz), 7.15 (d, 1H, *J*=1.6 Hz), 8.70 (d, 1H, *J*=9.8 Hz), 8.86 (d, 1H, *J*=9.8 Hz), 8.99 (d, 1H, *J*=8.8 Hz), 10.07 (s, 3H), 12.77 (s, 1H). Yield 93%

### Step iii: disodium 2-[({1-cyclopropyl-4-[(3-nitrobenzoyl)amino]-1H-pyrrol-2-yl}carbonyl)amino]naphthalene-1,5-disulfonate salt (29b)

A solution of 3-nitrobenzoyl chloride (140 mg, 0.75mmol) in 1,4-dioxane (15 mL) was added dropwise during 1 h to a cold (5 °C) solution of disodium 2-{[(4-amino-1-cyclopropyl-1*H-*pyrrol-2-yl)carbonyl]amino}naphthalene-1,5-disulfonate salt chloride (200 mg, 0.38 mmol) and sodium acetate (60 mg, 0.75 mmol) in water (15 mL). After stirring for 3 h at 5°C, the solution was acidified to pH 4 with HCl 1N and evaporated to dryness. The residue was treated with ethyl acetate (100 mL), stirred for 4 h and filtered to give disodium 2-[({1-cyclopropyl-4-[(3-nitrobenzoyl)amino]-1*H*-pyrrol-2-yl}carbonyl)amino]naphthalene-1,5-disulfonate salt 210 mg (85%) which was used in the next step without further purification.

¹H-NMR 200 MHz (DMSO-*d*₆) δ: 0.93-1.09 (m, 4H), 3.89-4.05 (m, 1H), 7.06 (s, 1H), 7.37-7.31 (m, 2H), 7.57 (s, 1H), 7.88-7.80 (m, 2H), 8.47-8.40 (m, 2H), 8.88-8.77 (m, 2H), 9.01 (d, 1H, *J*=8.6 Hz), 10.91 (s, 1H), 12.71 (s, 1H).

### Step iv: disodium 2-[({4-[(3-aminobenzoyl)amino]-1-cyclopropyl-1H-pyrrol-2-yl}carbonyl)amino]naphthalene-1,5-disulfonate salt (30b)

¹H-NMR 200 MHz (DMSO-*d*₆) δ: 0.90-1.10 (m, 4H), 3.84-4.04 (m, 1H), 5.27 (s, 2H), 6.63-6.74 (m, 1H), 6.99 (d, 1H, *J*=1.6 Hz), 7.09-7.12 (m, 3H), 7.50 (d, 1H, *J*=1.6 Hz), 7.83-7.72 (m, 1H), 8.71 (d, 1H, *J*=9.8 Hz), 8.84 (d, 1H, *J*=8.6 Hz), 8.96-9.02 (m, 1H), 10.29 (s, 1H), 12.63 (s, 1H).

### Step v: disodium 2,2'-{carbonylbis[imino-3,1-phenylenecarbonylimino(1-cyclopropyl-1H-pyrrole-4,2-diyl)carbonylimino]}dinaphthalene-1,5-disulfonate salt SST0591NA1 (31b)

¹H-NMR 400 MHz (DMSO-*d*₆) δ: 0.92-1.01 (m, 4H), 3.97-4.03 (m, 1H), 7.02 (s, 1H), 7.34-7.43 (m, 2H), 7.56-7.60 (m, 2H), 7.95 (s, 1H), 8.74 (d, 1H, *J*=9.6 Hz), 8.84 (d, 1H, *J*=10.0 Hz), 9.01 (d, 1H, *J*=8.8 Hz), 9.52 (s, 1H), 10.51 (s, 1H), 12.67 (s, 1H).

### Example 3

### Disodium 2,2'-{carbonylbis[imino-3,1-phenylenecarbonylimino(1,5-dimethyl-1H-pyrrole-4,2-diyl)carbonylimino]}dinaphthalene-1,5-disulfonate salt (SST0592NA1) (31c)

Compound **31c** was synthesized as described in example **1** using a different pyrrolic derivate in step i (1,5-dimethyl-4-nitro-1*H*-pyrrole-2-carbonyl chloride) and a different procedure in step iii (Scheme 5).

### Step i: disodium 2-{[(1,5-dimethyl-4-nitro-1H-pyrrol-2-yl)carbonyl]amino}naphthalene-1,5-disulfonate salt (26c)

¹H-NMR 200 MHz (DMSO-*d*₆) δ: 2.65 (s, 1H), 3.95 (s, 1H), 7.38-7.44 (m, 2H), 7.82-7.86 (m, 1H), 8.65 (d, 1H, *J*=9.6 Hz), 8.87 (d, 1H, *J*=9.6 Hz), 8.99 (d, 1H, *J*=8.4 Hz), 12.93 (s, 1H). Yield 82%

### Step ii: disodium 2-{[(4-amino-1,5-dimethyl-1H-pyrrol-2-yl)carbonyl]amino}naphthalene-1,5-disulfonate salt chloride (27b)

¹H-NMR 200 MHz (DMSO-*d*₆) δ: 2.29 (s, 3H), 3.86 (s, 3H), 6.90 (s, 1H), 7.36-7.41 (m, 1H), 7.83-7.80 (m, 1H), 8.66 (d, 1H, *J*=9.6 Hz), 8.83 (d, 1H, *J*=9.6 Hz), 8.99 (d, 1H, *J*=8.8 Hz), 10.16 (s,3H), 12.67 (s, 1H). Quantitative yield

### Step iii: disodium 2-[({1,5-dimethyl-4-[(3-nitrobenzoyl)amino]-1H-pyrrol-2-yl}carbonyl)amino]naphthalene-1,5-disulfonate salt (29c)

¹H-NMR 200 MHz (DMSO-*d*₆) δ: 2.18 (s, 3H), 3.93 (s, 3H), 7.07 (s, 1H), 7.46-7.54 (m, 2 H), 7.77-7.81 (m, 1H), 8.06-8.09 (m, 1H), 8.33-8.42 (m, 3H), 8.81-8.95 (m, 2H), 9.14 (d, 1H, *J*=8.8 Hz), 12.16 (s, 1 H). Yield 75%

### Step iv: disodium 2-[({4-[(3-aminobenzoyl)amino]-1,5-dimethyl-1H-pyrrol-2-yl}carbonyl)amino]naphthalene-1,5-disulfonate salt (30c)

¹H-NMR 200 MHz (DMSO-*d*₆) δ: 2.13 (s, 3H), 3.88 (s, 3H), 5.26 (s, 2H), 6.74-6.84 (m, 1H), 6.92 (s, 1H), 7.09-7.12 (m, 3 H), 7.34-7.38 (m, 1 H), 7.82-7.79 (m, 1 H), 8.71(d, 1H, *J*=9.6 Hz), 8.82 (d, 1H, *J*= 9.6 Hz), 8.99 (d, 1H, *J*=8.8 Hz), 9.51 (s, 1H), 12.55 (s, 1H). Yield 93%

### Step v: disodium 2,2'-{carbonylbis[imino-3,1-phenylenecarbonylimino(1,5-dimethyl-1H-pyrrole-4,2-diyl)carbonylimino]}dinaphthalene-1,5-disulfonate salt SST0592NA1 (31c)

¹H-NMR 400 MHz (DMSO-*d*₆) δ: 2.17 (s, 3H), 3.90 (s, 3H), 6.96 (s, 1H), 7.36-7.45 (m, 2H), 7.55-7.62 (m, 1H), 7.79-7.81 (m, 1H), 8.04 (s, 1H), 8.71 (d, 1H, *J*=9.6 Hz), 8.81 (d, 1H, *J*=9.6 Hz), 9.03 (d, 1H, *J*=8.8 Hz), 9.72 (s, 1H), 10.18 (s, 1H), 12.57 (s, 1H). Yield 15%

### Example 4

### Disodium 2,2'-{carbonylbis[imino(4-fluoro-3,1-phenylene)carbonylimino(1-methyl-1H-pyrrole-4,2-diyl)carbonylimino]}dinaphthalene-1,5-disulfonate salt (SST0612NA1) (31e)

Example 4 was synthesized following procedures described in Example 1 (step i to ii and iv to v) and in Example 2 (step iii) using the appropriate acid chloride derivate (3-nitro-5-(trifluoromethyl)benzoyl chloride).

### Step iii: disodium 2-[({4-[(4-fluoro-3-nitrobenzoyl)amino]-1-methyl-1H-pyrrol-2-yl}carbonyl)amino]naphthalene-1,5-disulfonate salt (29e)

¹H-NMR 400 MHz (DMSO-*d*₆) δ: 3.97 (s, 3H), 7.09 (d, 1H, *J*=1.6 Hz), 7.37 (m, 1H), 7.63 (d, 1H, *J*=1.6 Hz), 7.81-7.83 (m, 1H), 8.67-8.72 (m, 2H), 8.80-8.83 (m, 2 H), 9.01 (d, 1H, *J*=8.8 Hz), 9.09 (s, 1H), 11.12 (s, 1H), 12.76 (s, 1H). Yield 95%

### Step iv: disodium 2-[({4-[(3-amino-4-fluorobenzoyl)amino]-1-methyl-1H-pyrrol-2-yl}carbonyl)amino]naphthalene-1,5-disulfonate salt chloride (30e)

¹H-NMR 200 MHz (DMSO-*d*₆) δ: 3.99 (s, 3H), 7.03 (s, 1 H), 7.26-7.55 (m, 4 H), 7.79-7.83 (m, 2H), 8.71-9.03 (m, 3H), 10.54 (s, 1H), 12.69 (s, 1H). Quantitative yield.

### Step v: Disodium 2,2'-{carbonylbis[imino(4-fluoro-3,1-phenylene)carbonylimino(1-methyl-1H-pyrrole-4,2-diyl)carbonylimino]}dinaphthalene-1,5-disulfonate salt SST0612NA1 (31e)

¹H-NMR 400 MHz (DMSO-*d*₆) δ: 3.96 (s, 3H), 7.06 (d, 1H, *J*=1.2 Hz), 7.35-7.39 (m, 1H), 7.62 (d, 1H, *J*=1.2 Hz), 7.81-7.83 (m, 1H), 8.00 (s, 1H), 8.16 (s, 1H), 8.28 (s, 1H), 8.71 (d, 1H, *J*=9.6 Hz), 8.83 (d, 1H, *J*=9.6 Hz), 9.01 (d, 1H, *J*=8.8 Hz), 9.81 (s, 1H), 10.77 (s, 1H), 12.73 (s, 1 H). Yield 18%

### Example 5

### Disodium 2,2'-{carbonylbis[imino[5-(trifluoromethyl)-3,1-phenylene]carbonylimino(1-methyl-1H-pyrrole-4,2-diyl)carbonylimino]}dinaphthalene-1,5-disulfonate salt SST0613NA1 (31d)

Example 5 was synthesized following procedures described in Example 1 (step i to ii and iv to v) and in Example 2 (step iii) using the appropriate acid chloride derivate (4-fluoro-3-nitro-benzoyl chloride).

### Step iii: disodium 2-{[(1-methyl-4-{[3-nitro-5-(trifluoromethyl)benzoyl]amino}-1H-pyrrol-2-yl)carbonyl]amino}naphthalene-1,5-disulfonate salt (29d)

¹H-NMR 200 MHz (DMSO-*d*₆) δ: 3.95 (s, 3H), 7.06 (d, 1H, *J*=1.6 Hz), 7.36-7.37(m, 1H), 7.59 (d, 1H, *J*=1.6 Hz), 7.81-7.83 (m, 2H), 8.43-8.47 (m, 1H), 8.71 (d, 1H, *J*=9.2 Hz), 8.76-8.86 (m, 2H), 8.99 (d, 1H, *J*=8.8 Hz), 10.89 (s, 1H), 12.73 (s, 1 H). Yield 87 %

### Step iv: disodium 2-{[(4-{[3-amino-5-(trifluoromethyl)benzoyl]amino}-1-methyl-1H-pyrrol-2-yl)carbonyl]amino}naphthalene-1,5-disulfonate salt chloride (30d)

¹H-NMR 400 MHz (DMSO-*d*₆) δ: 3.92 (s,3H), 7.01 (s, 1H), 7.09-7.22 (m, 2H), 7.34-7.43 (m, 2H), 7.53 (s, 1H), 7.80-7.82(m, 1H), 8.69 (d, 1H, *J*=9.6 Hz), 8.83 (d, 1H, *J*=9.6 Hz), 9.00 (d, 1H, *J*=8.8 Hz), 10.37 (s, 1H), 12.66 (s, 1H).

### Step v: disodium 2,2'-{carbonylbis[imino[5-(trifluoromethyl)-3,1-phenylene]carbonylimino(1-methyl-1H-pyrrole-4,2-diyl)carbonylimino]}dinaphthalene-1,5-disulfonate salt SST0613NA1 (31d).

¹H-NMR 400 MHz (DMSO-*d*₆) δ: 3.93 (s, 3H), 7.02 (d, 1H, *J*=1.6 Hz), 7.32-7.42 (m, 3H), 7.58 (d, 1H, *J*=1.6 Hz), 7.69-7.73 (m, 1H), 7.79-7.81 (m, 1H), 8.68-8.83 (m, 3H), 8.98-9.00 (m, 1H), 10.52 (s, 1H), 12.68 (s, 1H). Yield 21%

### EXAMPLES 6-7

The compounds **37 a,b** where the second pyrrole was replaced by benzoic acid and 4-methyl benzoic acid residues were synthesized as described in Scheme 7. The condensation of 2-amino naphthalene-1,5-disulfonic acid disodium salt **1** with 3-nitro-benzoyl-chloride **28 a** or 4-methyl-3-nitro-benzoyl-chloride **32** gives the nitro derivatives **33 a,b** which were easily reduced by catalytic hydrogenation to amines **34 a,b** in quantitative yield. The latter amines reacted with 1-methyl-4-nitro-pyrrole-2-carbonyl chloride **2** to give the nitro derivatives **35 a,b** in a yield between 70 and 80%. The final condensation reaction to obtain the urea derivatives **37 a,b** was possible by means of the phosgene as condensation agent in a yield between 10 of 20% using the amines **36 a,b** which were obtained using catalytic hydrogenation in quantitative yield.

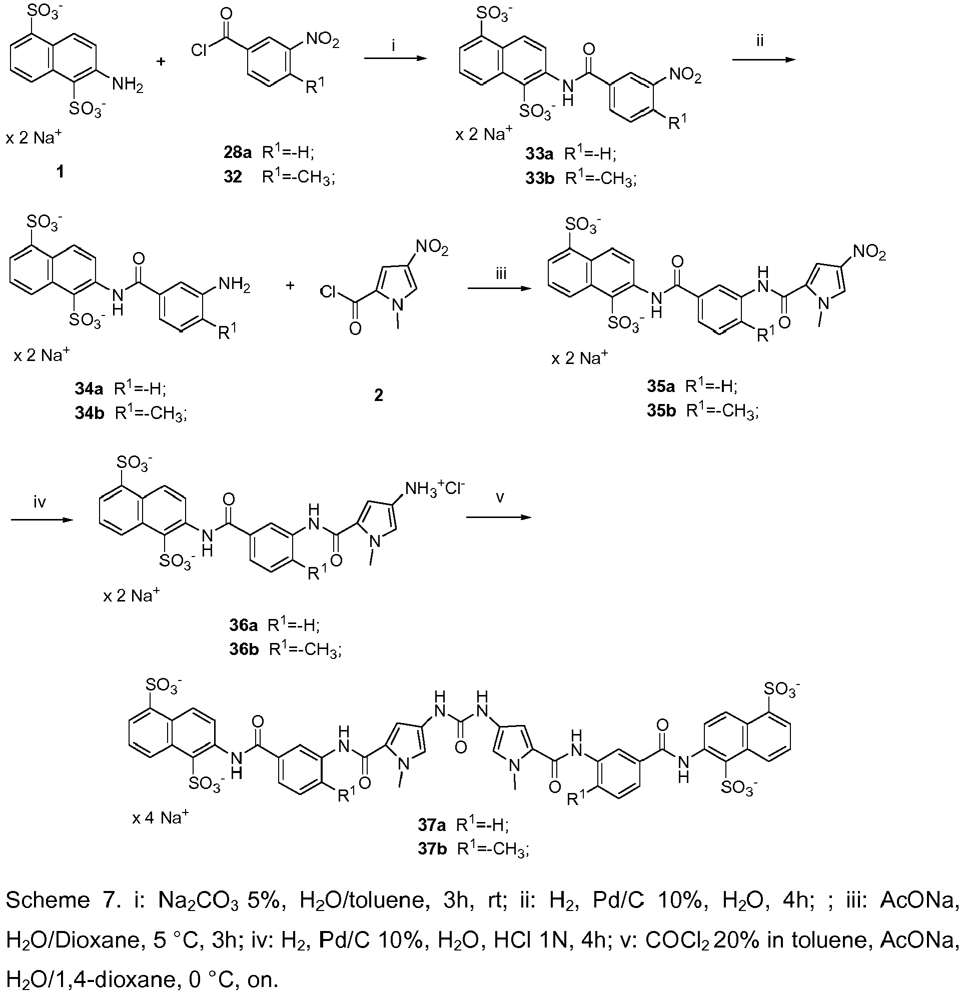

### Example 6

### Disodium 2,2'-{carbonylbis[imino(1-methyl-1H-pyrrole-4,2-diyl)carbonylimino-3,1-phenylenecarbonylimino]}dinaphthalene-1,5-disulfonate salt SST0546NA1 (37a)

Example 6 was synthesized following the procedures described in Scheme 8.

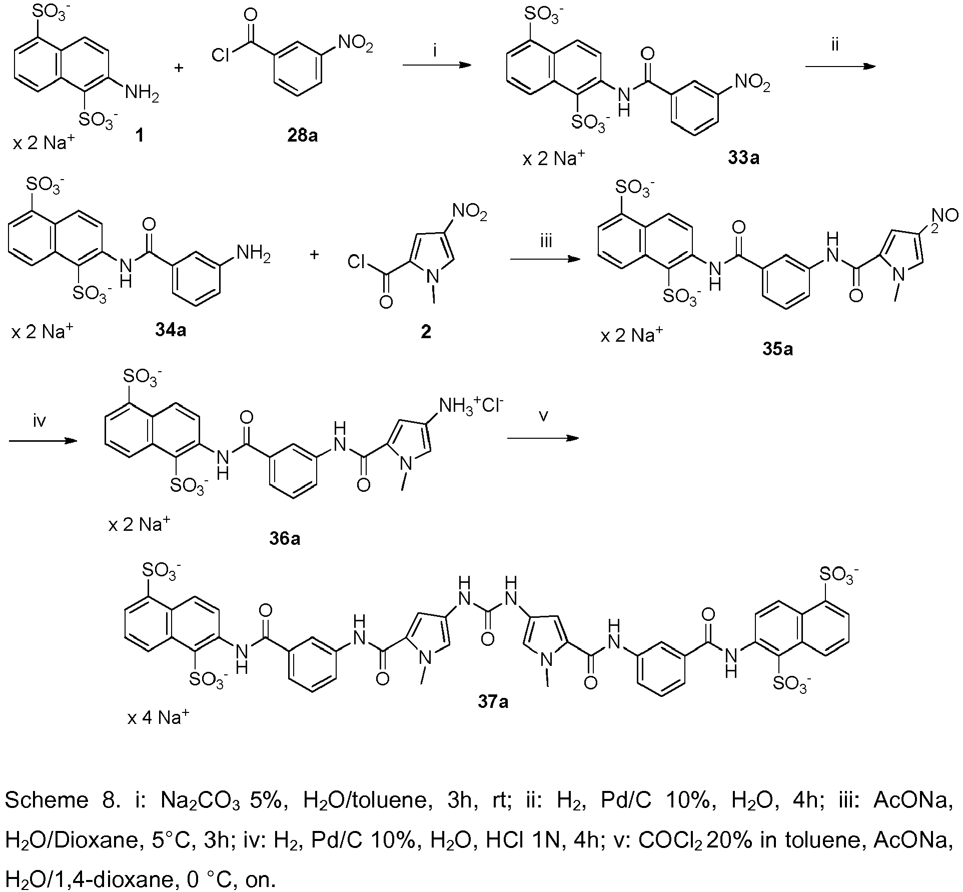

### Step i: disodium 2-[(3-nitrobenzoyl)amino]naphthalene-1,5-disulfonate salt

Disodium 2-aminonaphthalene-1,5-disulfonic acid (690 mg, 1.99 mmol) was dissolved in H₂O (20 mL) and the pH was adjusted to 3.8. To the stirred solution 3-nitrobenzoyl chloride (550 mg, 2.99 mmol) dissolved in 7 mL of toluene was slowly added. The reaction mixture was kept at a costant pH of 3.8 by addition of a 5% solution of Na₂CO₃ in H₂O. After separating the toluene from the water phase, pH was adjusted to 2.0 and the aqueous phase was extracted four times with Et₂O 8 mL, respectively. After neutralisation (pH 7.0), the water was removed under vacuum to give disodium 2-[(3-nitrobenzoyl)amino]naphthalene-1,5-disulfonate salt 990 mg (82%).

¹H-NMR 200 MHz (DMSO-*d*₆) δ: 7.38-7.46 (m, 1H), 7.86-7.96 (m, 2H), 8.41-8.47 (m, 2H), 8.73-8.80 (m, 2H), 8.93 (d, 1H, *J*=9.6 Hz), 9.03 (d, 1H, *J*=8.8 Hz), 13.39 (s, 1H).

### Step ii: disodium 2-[(3-aminobenzoyl)amino]naphthalene-1,5-disulfonate salt (34a)

A solution of disodium 2-[(3-nitrobenzoyl)amino]naphthalene-1,5-disulfonate salt (990 mg, 1.9 mmol) in H₂O (20 mL) was hydrogenated over 10% Pd/C (catalytic amount) for 4h. The catalyst was filtered off and the resulting solution was concentrated to afford disodium 2-[(3-aminobenzoyl)amino]naphthalene-1,5-disulfonate salt 600 mg (98,8%).

¹H-NMR 400 MHz (DMSO-*d*₆) δ: 5.35 (s, 2H), 6.75-6.79 (m, 1H), 7.14-7.21(m, 3H), 7.33-7.41 (m, 1H), 7.81-7.85 (m, 1H), 8.69 (d, 1H, *J*=9.6 Hz), 8.86 (d, 1H, *J*=9.6 Hz), 9.01 (d, 1H, *J*=8.8 Hz), 12.81 (s, 1H).

### Step iii: disodium 2-[(3-{[(1-methyl-4-nitro-1H-pyrrol-2-yl)carbonyl]amino}benzoyl)amino]naphthalene-1,5-disulfonate salt (35a)

A solution of 1-methyl-4-nitro-1*H*-pyrrole-2-carbonyl chloride (160 mg, 0.85mmol) in 1,4-dioxane (10 mL) was added dropwise (1 h) to a cold solution (5 °C) of disodium 2-[(3-aminobenzoyl)amino]naphthalene-1,5-disulfonate salt (200 mg, 0.43 mmol) and sodium acetate (70 mg, 0.85 mmol) in H₂O (10 mL). After stirring for 3 h at 5 °C, the solution was acidified to pH 4 with 1N HCl and evaporated to dryness. The residue was treated with EtOAc (100 mL), stirred for 4 h and filtered to give disodium 2-[(3-{[(1-methyl-4-nitro-1*H-*pyrrol-2-yl)carbonyl]amino}benzoyl)amino]naphthalene-1,5-disulfonate salt 220 mg (85%) which was used in the next step without further purification.

¹H-NMR 200 MHz (DMSO-*d*₆) δ: 3.99 (s, 3H), 7.35-7.43 (m, 1H), 7.49-7.58 (m, 1H), 7.69-7.74 (m, 1H), 7.83-7.88 (m, 2H), 8.01-8.06 (m, 1H), 8.25-8.26 (m, 1H), 8.39 (m, 1H), 8.71 (d, 1H, *J*=9.6 Hz), 8.89 (d, 1H, *J*=9.6 Hz), 9.02 (d, 1H, *J*=8.8 Hz), 10.48 (s, 1H), 13.02 (s, 1H).

### Step iv: disodium 2-[(3-{[(4-amino-1-methyl-1H-pyrrol-2-yl)carbonyl]amino}benzoyl)amino]naphthalene-1,5-disulfonate chloride (36a)

A solution of disodium 2-[(3-{[(1-methyl-4-nitro-1*H*-pyrrol-2-yl)carbonyl]amino}benzoyl)amino]naphthalene-1,5-disulfonate salt (220 mg, 0.36 mmol) in H₂O (20 mL) and 1 N HCl (pH= 3) was hydrogenated over 10% Pd/C (catalytic amount) for 4h. The catalyst was filtered off and the resulting solution was concentrated to afford disodium 2-[(3-{[(4-amino-1-methyl-1*H*-pyrrol-2-yl)carbonyl]amino}benzoyl)amino]naphthalene-1,5-disulfonate chloride 220 mg. Quantitative yield.

¹H-NMR 200 MHz (DMSO-*d*₆) δ: 3.92 (s, 3H), 7.35-7.57 (m, 4H), 7.67-7.73 (m, 1H), 7.83-7.87 (m, 1H), 8.03-8.11 (m, 1H), 8.33 (s, 1H), 8.71 (d, 1H, *J*=9.6 Hz), 8.89 (d, 1 H, *J*=9.6 Hz), 9.02 (d, 1H, *J*=8.8 Hz), 10.19 (s, 1H) 10.22 (s, 3H), 13.01 (1H).

### Step v: disodium 2,2'-{carbonylbis[imino(1-methyl-1H-pyrrole-4,2-diyl)carbonylimino-3,1-phenylenecarbonylimino]}dinaphthalene-1,5-disulfonate salt SST0546NA1 (37a)

A 20% solution of phosgene in toluene (0.5 mL, 0.90 mmol), diluited with 1,4-dioxane (1.5 mL), was added slowly to a cold solution (5 °C) of disodium 2-[(3-{[(4-amino-1-methyl-1*H-*pyrrol-2-yl)carbonyl]amino}benzoyl)amino]naphthalene-1,5-disulfonate chloride (280 mg, 0.45 mmol) and sodium acetate (150 mg, 1.8 mmol) in H₂O (15 mL) and 1,4-dioxane (4 mL). After stirring for 2 h at 5 °C, solvents were evaporated under vacuum and the residue was taken up in MeOH. Precipitated salts were filtered off, the filtrate was evaporated and the residue was purified by column chromatography on a silica gel column with CH₂Cl₂:MeOH:H₂O (60:40:4 v/v) as eluent to give disodium 2,2'-{carbonylbis[imino(1-methyl-1*H*-pyrrole-4,2-diyl)carbonylimino-3,1-phenylenecarbonylimino]}dinaphthalene-1,5-disulfonate salt SST0546NA1 42 mg (10%).

¹H-NMR 400 MHz (DMSO-*d*₆) δ: 3.87 (s, 3H), 6.97 (d, 1H, *J*=1.6 Hz), 7.16 (d, 1H, *J*=1.6 Hz), 7.37-7.41 (m, 1H), 7.48-7.52 (m, 1H), 7.65-7.67 (m, 1H), 7.83-7.86 (m, 1H), 8.02-8.04 (m, 1H), 8.22 (s, 1H), 8.35-8.36 (m, 1H), 8.71 (d, 1H, *J*= 9.6 Hz), 8.89 (d, 1H, *J*=9.6 Hz), 9.01 (d, 1H, *J*=8.8 Hz), 9.97 (s, 1H), 12.99 (s, 1H).

### Example 7

### Disodium 2,2'-{carbonylbis[imino(1-methyl-1H-pyrrole-4,2-diyl)carbonylimino(4-methyl-3,1-phenylene)carbonylimino]}dinaphthalene-1,5-disulfonate salt SST0562NA1 (37b)

Example 7 was synthesized using the same procedures followed for example 6 using the appropriate acid chloride in step i (4-methyl-3-nitrobenzoyl chloride).

### Step i: disodium 2-[(4-methyl-3-nitrobenzoyl)amino]naphthalene-1,5-disulfonate salt (33b)

¹H-NMR 200 MHz (DMSO-*d*₆) δ: 2.63 (s, 3H), 7.36-7.44 (m, 1H), 7.74-7.88 (m, 2H), 8.19-8.24 (m, 1H), 8.56-8.58 (m, 1H), 8.74 (d, 1H, *J*=9.6 Hz), 8.91 (d, 1H, *J*=9.6 Hz), 9.01 (d, 1H, *J*=8.8 Hz), 13.31 (s, 1H).

### Step ii: disodium 2-[(3-amino-4-methylbenzoyl)amino]naphthalene-1,5-disulfonate salt (34b)

¹H-NMR 200 MHz (DMSO-*d*₆) δ: 2.13 (s, 3H), 5.10 (s, 2H), 7.08-7.10 (m, 2H), 7.27-7.40 (m, 2H), 7.80-7.84 (m, 1H), 8.69 (d, 1H, *J*=9.6 Hz), 8.85 (d, 1H, *J*=9.6 Hz), 9.00 (d, 1H, *J*=8.8 Hz), 12.78 (s,1H).

### Step iii: disodium 2-[(4-methyl-3-{[(1-methyl-4-nitro-1H-pyrrol-2-yl)carbonyl]amino}benzoyl)amino]naphthalene-1,5-disulfonate salt (35b)

¹H-NMR 200 MHz (DMSO-*d*₆) δ: 2.32 (s, 3H), 3.97 (s, 3H), 7.34-7.50 (m, 2H), 7.75-7.95 (m, 4H), 8.25 (d, 1H, *J*=2.0 Hz), 8.75 (d, 1H, *J*=9.6 Hz), 8.88 (d, 1H, *J*=9.6 Hz), 9.50 (d, 1H, *J*=8.8 Hz), 10.11 (s, 1H), 13.06 (s, 1H).

### Step iv: disodium 2-[(3-{[(4-amino-1-methyl-1H-pyrrol-2-yl)carbonyl]amino}-4-methylbenzoyl)amino]naphthalene-1,5-disulfonate salt chloride (36b)

¹H-NMR 200 MHz (DMSO-*d*₆) δ: 2.30 (s, 3H), 3.89 (s, 3H), 7.15-7.21 (m, 2H), 7.39-7.47 (m, 2H), 7.72-7.92 (m, 3H), 8.83 (d, 1 H, J=9.6 Hz), 8.88 (d, 1 H, *J*=8.8 Hz), 9.91 (s, 1 H), 10.35 (s, 3H), 13.04 (s, 1 H).

### Step v: disodium 2,2'-{carbonylbis[imino(1-methyl-1H-pyrrole-4,2-diyl)carbonylimino(4-methyl-3,1-phenylene)carbonylimino]}dinaphthalene-1,5-disulfonate salt SST0562NA1 (37b)

¹H-NMR 400 MHz (DMSO-*d*₆) δ: 2.31 (s, 3H), 3.87(s, 3H), 6.91 (d, 1H, *J*=1.6 Hz), 7.13 (d, 1H, *J*=1.6 Hz), 7.36-7.46 (m, 2H), 7.81-7.98 (m, 3H), 8.36 (s, 1H), 8.76 (d, 1H, *J*=9.6 Hz), 8.88 (d, 1H, *J*=9.6 Hz), 9.01 (d, 1H, *J*=8.8 Hz), 9.58 (s, 1 H), 13.04 (s, 1H).

### Example 8

### Synthesis of FCE27266

### Disodium 2,2'-{carbonylbis[imino(1-methyl-1H-pyrrole-4,2-diyl) carbonylimino(1-methyl-1H-pyrrole-4,2-diyl)carbonylimono]}dinaphthalene-1,5-disulfonate salt - FCE27266 (SST0533NA1)

FCE27266 was synthesized following the procedures described in following scheme 9.

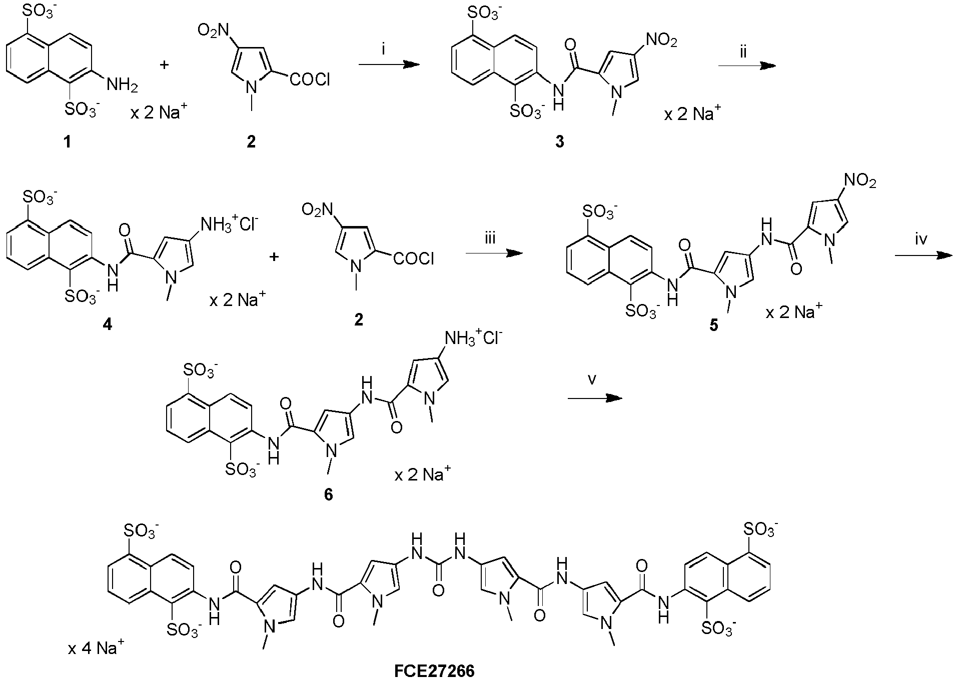

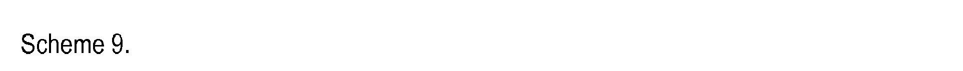

### Step i: 2-(1-Methyl-4-nitro-1H-pyrrole-2-carboxamido)naphthalene-1,5-disulfonic acid disodium salt (3)

A solution of 1-methyl-4-nitro-1*H*-pyrrole-2-carbonyl chloride (950 mg, 5 mmol) in 1,4-dioxane (45 mL) was added dropwise (1 h) to a cold solution (5 °C) of disodium-2-amino naphthalene-1,5-disulfonate salt (1.03 g, 2.96 mmol) and sodium acetate (490 mg, 5.93 mmol) in water (45 mL). After stirring for 3 h at 5 °C, the solution was acidified to pH 4 with HCl 1N and evaporated to dryness. The residue was treated with ethyl acetate (300 mL), stirred for 4 h and filtered to give 2-(1-Methyl-4-nitro-1H-pyrrole-2-carboxamido)naphthalene-1,5-disulfonic acid disodium salt 1.34 g (91%), which was used in the next step without further purification.

¹H-NMR 400 MHz (DMSO-*d*₆) δ: 4.03 (s, 3H), 7.38-7.40 (m, 2H), 7.86 (d, 1H, *J*=6,8 Hz), 8.29 (d, 1H, *J*=1.2 Hz), 8.65 (d, 1H, *J*=9.6 Hz), 8.87 (d, 1H, J=9.6 Hz), 9.00 (d, 1H, *J*=8.8 Hz), 13.00 (s, 1H).

### Step ii: disodium 2-{[(4-amino-1-methyl-1H-pyrrol-2-yl)carbonyl]amino}naphthalene-1,5-disulfonate salt chloride (4)

To a solution of disodium-2-(1-Methyl-4-nitro-1H-pyrrole-2-carboxamido)naphthalene-1,5-disulfonate salt (600 mg, 1.2 mmol) in water (40 mL) was added 1N HCl (1.2 mmol, 1.2 mL) (pH= 3) and was hydrogenated over 10% Pd/C (catalytic amount) for 4h. The catalyst was filtered off and the resulting solution was concentrated to afford disodium 2-{[(4-amino-1-methyl-1*H*-pyrrol-2-yl)carbonyl]amino}naphthalene-1,5-disulfonate salt chloride 600 mg (98.8%).

¹H-NMR 400 MHz (DMSO-*d*₆) δ: 3.94 (s, 3H), 6.89 (d, 1H, *J*=2.0 Hz), 7.19 (d, 1H), 7.33-7.37 (m, 1H), 7.80 (d, 1H, *J*=6.0 Hz), 8.66 (d, 1H, *J*=9.6 Hz), 8.82 (d, 1H, J=10.0 Hz), 8.97(d, 1H, *J*=9.2 Hz), 9.95 (s, 3H), 12.79 (s, 1 H).

### Step iii: Disodium 2-{[(1-methyl-4-{[(1-methyl-4-nitro-1H-pyrrol-2-yl)carbonyl]amino}-1H-pyrrol-2-yl)carbonyl]amino}naphthalene-1,5-disulfonate salt (5) was synthesized following procedure described in Step i.

¹H-NMR 200 MHz (DMSO-*d*₆) δ: 3.92 (s, 3H), 3.98 (s, 3H), 7.01 (d, 1H), 7.36-7.40 (m, 1H), 7.48 (d,1H), 7.68 (d, 1H, *J*=1.8 Hz), 7.82 (d, 1H, *J*=6.4 Hz), 8.20 (d, 1H, *J*=1.4 Hz), 8.70 (d, 1H, *J*=9.8 Hz), 8.84 (d, 1H, *J*=9.8 Hz), 9.00 (d, 1H, *J*=8.4 Hz), 10.49(s, 1H), 12.69(s, 1H). Yield 67%.

### Step iv: disodium-2-{[(4-{[(4-amino-1-methyl-1H-pyrrol-2-yl)carbonyl]amino}-1-methyl-1H-pyrrol-2-yl)carbonyl]amino}naphthalene-1,5-disulfonate salt chloride (6) was synthesized following procedure described in Step ii.

¹H-NMR 400 MHz (CD₃OD) δ: 3.89 (s, 3H), 3.94 (s, 3H), 6.93 (s, 1H), 6.95 (s, 1H), 7.00 (s, 1H), 7.35 (s, 1H), 7.48-7.52 (m, 1H), 8.07-8.08 (m, 1H), 8.82 (d, 1H, *J*=9.6 Hz), 8.92 (d, 1H, *J*=9.6 Hz), 9.14 (d, 1H, J=8.8 Hz). Yield 97%.

### Step v: Disodium 2,2'-{carbonylbis[imino(1-methyl-1H-pyrrole-4,2-diyl) carbonylimino(1-methyl-1H-pyrrole-4,2-diyl)carbonylimino]}dinaphthalene-1,5-disulfonate salt

A 20% solution of phosgene in toluene (1 mL, 2 mmol), diluited with 1,4-dioxane (3 mL), was added slowly to a cold solution (5 °C) of disodium 2-{[(4-{[(4-amino-1-methyl-1*H*-pyrrol-2-yl)carbonyl]amino}-1-methyl-1*H*-pyrrol-2-yl)carbonyl]amino}naphthalene-1,5-disulfonate hydrochloride (640 mg, 1.02 mmol) and sodium acetate (330 mg, 4 mmol) in water (20 mL) and 1,4-dioxane (5 mL). After stirring for 2 h at 5 °C, solvents were evaporated under vacuum and the residue was taken up in MeOH. Precipitated salts were filtered off, the filtrate was evaporated and the residue was purified by column chromatography on a silica gel with CH₂Cl₂:MeOH:H₂O (60:40:4 v/v) as eluent to give Disodium 2,2'-{carbonylbis[imino(1-methyl-1*H*-pyrrole-4,2-diyl) carbonylimino(1-methyl-1*H*-pyrrole-4,2-diyl)carbonylimino]}dinaphthalene-1,5-disulfonate salt (55%).

### Biological assays

### Example 9

### In vitro screening for heparanase activity

All compounds were tested in a simple, accurate and robust microplate assay based on the cleavage of the synthetic heparin fragment, the pentasaccharide Fondaparinux (AGA*IA). This assay was originally developed by Hammond and coworkers [Hammond E, Li CP, Ferro V. Development of a colorimetric assay for heparanase activity suitable for kinetic analysis and inhibitor screening. Anal. Biochem. 396(1), 112-116 (2010)], where Fondaparinux corresponds to the methyl glycoside of the antithrombin III (ATIII)-activating pentasaccharide sequence of heparin. Fondaparinux is cleaved by heparanase to generate a reducing disaccharide, which can be quantitatively measured via a colorimetric or a fluorescence assay, the last by the fluorescent sensor polymer-H [Schiemann S, Lühn S, Alban S. Development of both colorimetric and fluorescence heparinase activity assays using fondaparinux as substrate. Anal. Biochem. 427(1), 82-89 (2012)].

### Materials and methods

To determine the activity of the newly designed heparanase inhibitors an homogenous assay based on the cleavage of the synthetic heparin oligosaccharide fondaparinux has been employed (Hammond E. et al. Anal. Biochem. 2010, 396, 112). The assay measures the appearance of the disaccharide product of heparanase-catalyzed fondaparinux cleavage colorimetrically using the tetrazolium salt WST-1. Assay was essentially performed as described with minor modifications. Briefly, NUNC ELISA 96-well plates were pre-treated with a solution of 4% BSA (bovin serum albumin) in phosphate-buffered saline containing 0.05% Tween 20 (PBST), for 2 h at 37°C, then washed three times with PBST, dried by tapping on paper towel and stored at -20°C. Before use, once again, the plates were washed with PBST.

Assay was carried out with 100 µl/well of assay solution containing 40 mM sodium acetate buffer (pH 5.0), 100 µM fondaparinux, 2.5 nM heparanase and serial dilutions of test compounds (tested in triplicate). Plates were sealed with adhesive tape and incubated, in the dark, for 3 h at 37°C, followed by development with 1.69 mM of the tetrazolium salt WST-1 solution in 0.1 M NaOH, for 1 h at 60°C. Then, the absorbance at 560 nm was measured through a microplate reader (Victor 3, Perkin Elmer).

IC50 value for each compound, versus heparanase, was calculated by GraphPad software and results of selected compounds are summarized in the Table 2 below. Finally, the measurements were corrected by subtracting both the reagent background and the inner absorbance value of test compound.

### Results

Results of selected compounds are summarized in the Table 2 below.

**Table 2. Results of Heparanase inhibition activity of compounds of examples 1-7 of present invention**

| Example n. | Internal Code | AGAIA assay; IC50(µM) |
|---|---|---|
| 1 | SST0548NA1 | +++ |
| 2 | SST0591NA1 | ++ |
| 3 | SST0592NA1 | + |
| 4 | SST0612NA1 | + |
| 5 | SST0613NA1 | +++ |
| 6 | SST0546NA1 | ++ |
| 7 | SST0562NA1 | + |
| FCE27266 | SST0533NA1 | ∼ 2 |
| Reference | Suramin | ∼ 26 |

| | | |
|---|---|---|
| Legend: +++ (0.50-0.99 µM) ++ (1.00-2.49 µM) + (2.50-10.00 µM) | | |

FCE27266 showed a potent anti-heparanase activity, as shown in Table 2 above.

The new compounds of examples 1-7 showed an anti-heparanase activity in the AGA*IA assay in the same range of FCE27266 and more potent than suramin, the latter being a reference compound for heparanase inhibition activity.

Upon screening for inhibition of heparanase enzymatic activity, selected active compound were further characterized *in vitro*, through a suitable cell proliferation assay (SRB Cell Cytotoxicity Assay) for their putative cytotoxic activity against three tumor cell lines, and through specific cellular assay for their putative anti-metastatic activity. According to these data selected compounds were also tested for the effects on the expression of proangiogenic factors by real-time quantitative PCR.

### Example 10

### Cytotoxicity, invasion and cell adhesion assays

### Cytotoxicity assay

SRB Cell Cytotoxicity Assay is an assay based upon the quantitative staining of cellular proteins by sulforhodamine B (SRB). This assay can be used for high-throughput drug screening (Vichai & Kirkitara, Nature Protocol. 2006, 1, 112). Sulforhodamine B is an anionic aminoxanthene dye that forms an electrostatic complex with the basic amino acid residues of proteins under moderately acid conditions, which provides a sensitive linear response.

### Materials and methods

To assess a putative antiproliferative activity of the selected compounds, HT1080 (human fibrosarcoma), U87MG (human glioblastoma astrocytoma) and U2OS (human osteosarcoma) cell lines were treated for 72h, in triplicate, with increasing concentrations of heparanase inhibitors. Inhibition of cell proliferation was then measured by the classical colorimetric SRB Cell Cytotoxicity assay. Absorbance was measured by spectrophotometer at a wavelength of 510 nm and cell proliferation was determined as percent with respect to control cells (cells treated with the vehicle alone). EC₅₀ values were then determined by GraphPad Prism.

The following Table 3 reports the percentage of tumor growth inhibition (% TGI) versus control of the tested compounds. Suramin was used as reference compound.

**Table 3. Putative anti-proliferative activity of the selected compounds on three tumor cell lines.**

| | | Concentration of test compounds at which the cell growth is inhibited by 50 % | | |
|---|---|---|---|---|
| | | HT1080 | U87MG | U2OS |
| *Reference compound* | Suramin | > 2.5 µM | > 2.5 µM | > 2.5 µM |
| FCE27266 | SST0533NA1 | > 2.5 µM | > 2.5 µM | > 2.5 µM |
| 31a | SST0548NA1 | > 2.5 µM | > 2.5 µM | > 2.5 µM |
| 31b | SST0591NA1 | > 2.5 µM | > 2.5 µM | > 2.5 µM |
| 31d | SST0613NA1 | > 2.5 µM | > 2.5 µM | > 2.5 µM |
| 37a | SST0546NA1 | > 2.5 µM | > 2.5 µM | > 2.5 µM |

All selected compounds, as well as reference compound (suramin), tested up to 2.5 µM, do not interfere with the cell growth on the three tumor cell lines. These data are very important because they show that some compounds of the present invention, at concentrations in which they do not have any interference with cell proliferation, are selective heparanase inhibitors. This makes their use particularly advantageous in conditions wherein an anti-heparanase activity is desired but a cytotoxic effect is undesirable.

Selected compounds were also tested in an invasion assay as well as in cell adhesion assay.

### The Invasion Assay

Heparanase inhibitors were then evaluated for their activity on the invasive potential of HT1080 (human fibrosarcoma) human cell lines, using the Matrigel cell invasion assay which allows to assess the cell invasion ability of malignant and normal cells.

### Materials and methods

Invasion assay was performed using BioCoat cell culture inserts (BioCoat 8.0µm PET Membrane 24 Well Permeable Supports, Corning). Filters were coated with 100 µl of 300 µg/ml Matrigel dissolved in coating buffer (0.01 M Tris pH 8.0, 0.7% NaCl) (Becton-Dickinson Sciences). DMEM (Dulbecco's Modified Eagle's Medium) containing 5% FBS was added to the lower chambers. Cells were pre-treated with heparanase inhibitors for 24 h, detached and added to the upper chambers in media containing the inhibitors. Cells were allowed to invade for 24 h at 37°C in a CO₂ incubator and then unmigrated cells were removed from the upper chamber with a cotton swab. The invading cells were fixed, stained with 0.1% crystal violet for 10 min at room temperature, photographed and counted under inverted microscope.

Test compounds were analyzed at fixed concentration for several fields, in duplicates. Activity of test compounds was compared to the reference compound. Activity of most interesting compound was confirmed in a second cell invasion assay. Data were expressed as the percent invasion with respect to cell migration through uncoated insert membrane. (Table 4)

### Cell Adhesion Assay

Cell surface, non-integrin molecules such as heparan sulfate proteoglycans (HSPGs) may be involved in the regulation of early adhesive stages of cell-ECM interactions. A cell adhesion assay has been employed to determine if the newly designed heparanase inhibitors were able to interfere with adhesion properties of cancer cells.

### Materials and methods

HT1080 (human fibrosarcoma) cells were treated for 18-20 h with test compounds in complete medium supplemented with 10% FCS. Then cells were collected, washed in complete medium, and added to matrigel coated wells in triplicate, at previously identified optimal seeding density, and incubated at 37 °C in complete medium for times ranging from 15 minutes to 4h, according to the cell line. After incubation, the wells were washed three times with serum-free medium and the remaining firmly attached cells were stained with crystal-violet 0.1%. Optical density was measured at 560 nm by Victor 3 (Perkin-Elmer) plate analyzer. Each experiment was repeated three times.

### Results

Results of the invasion and adhesion assays are reported in Table 4 below.

| Compound | Code lab | **Invasion Assay Inhibition** HT1080; 2.5 µM | **Adhesion Assay (%)** HT1080; 2.5 µM |
|---|---|---|---|
| Suramin | | <50 % | ∼ 100 |
| FCE27266 | SST0533NA1 | *N. D*. | ∼ 93 |
| 31a | SST0548NA1 | *N. D.* | ∼ 86 |
| 31b | SST0591NA1 | *N. D.* | ∼ 88 |
| 37a | SST0546NA1 | > 90 % | ∼ 86 |

| | | | |
|---|---|---|---|
| N.D. not determined | | | |

Table 4. Test compounds were analyzed at fixed concentration (2.5 µM) versus Reference compound (Suramin) at 10 µM. In Invasion assay, data were expressed as the percent invasion (range) with respect to cell migration through uncoated insert membrane. In Adhesion assay, data were expressed as the percent of adhesion with respect to the control (100%).

In the Invasion Assay SST0546NA1 has showed strong inhibition of invasion (> 90%) at 2.5 µM, concentration not cytotoxic and active on heparanase. In cell adhesion assay, at the same concentration, all compounds tested showed a weak Inhibition.

### Example 11

### Real-Time Quantitative PCR (qPCR) for measuring mRNA gene-expression of FGF1/2, VEGF, MMP-9, HSPE-1 in genes tumor cell lines

### Materials and methods

To evaluate the effect of heparanase inhibitors on the tumor transcription of genes encoding for proangiogenic factors, such as FGF1/2, VEGF, MMP-9, HSPE-1, the relative mRNA levels were analyzed by quantitative Real-Time Quantitative PCR. To perform the assay, HT1080 (human fibrosarcoma) cells were cultured in complete medium containing 10%FCS (fetal calf serum). Cells were plated in 24-well plates and treated at fixed concentration (i.e., highest non-toxic concentration) of test compounds. After 24 h, cells were detached, collected and total RNA extracted by RNeasy Mini Kit (QIAGEN). Following reverse transcription step, FGF1/2, VEGF, MMP-9, HSPE-1 mRNA levels, were quantified by qPCR using the advanced Universal SYBR Green Supermix (Bio Rad) and ABI PRISM 7900HT Thermal Cycler System (Perkin Elmer), according to the manufacturer's instructions. Appropriate no-RT and non-template controls were included in each 96-well PCR reaction, and dissociation analysis was performed at the end of each run to confirm the specificity of the reaction. Relative levels of RNA were calculated using the ddCt method. Standard curve and sample data were run in duplicate. In the Table a mean value is reported.

### Results

Results are reported in the following table 5.

**Table 5. Real-Time RT-PCR of pro-angiogenic genes**

| | | Gene Expression (% mRNA vs. control) | | | | |
|---|---|---|---|---|---|---|
| | Conc | FGF-1 | FGF-2 | VEGF | MMP-9 | HPSE-1 |
| Control | | ∼ 100 | ∼ 100 | ∼ 100 | ∼ 100 | ∼ 100 |
| SST0533 | 2.5 uM | ∼ 44 | ∼ 86 | ∼ 100 | ∼ 100 | ∼ 1 |
| SST0546 | 2.5 uM | ∼ 87 | ∼ 98 | ∼ 85 | ∼ 80 | ∼ 4 |
| SST0001 | 10.0 uM | ∼ 70 | ∼ 77 | ∼ 43 | ∼ 42 | ∼ 70 |

Data from qPCR assay, with two selected active compounds versus SST0001 (reference compound) on HT1080 (human fibrosarcoma) cells, highlighted an inhibitory effect against the tumor transcription genes encoding for nearly all proangiogenic factors tested.

SST0533NA1 and SST0546NA1, the two selected compounds, tested at the concentration of 2.5µM showed almost complete silencing of heparanase gene. They are also more potent than SST0001 tested at 10 µM.

## Claims

1. A compound having the following formula (I) 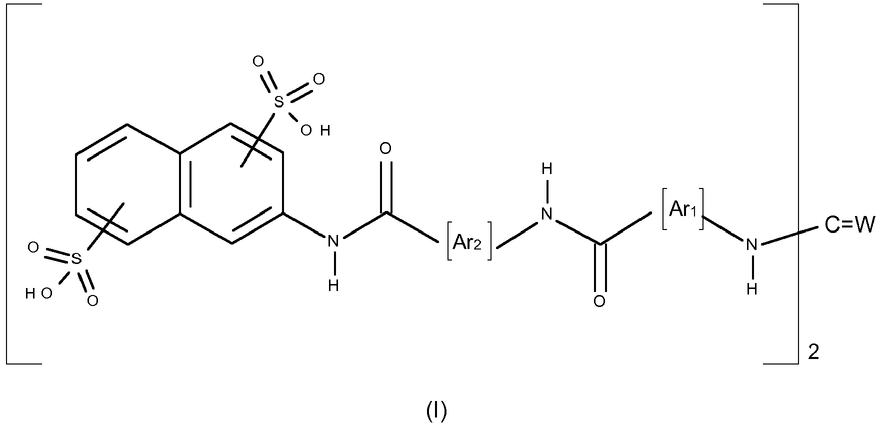 wherein
Ar₁ and Ar₂ can be the same or different and they are independently selected from the group consisting of: 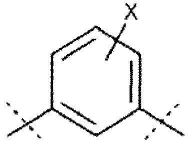
wherein X is selected from the group consisting of H, C₁-C₄alkyl, linear or branched, halogen and C₁-C₄alkyl substituted with one or more halogens;
and 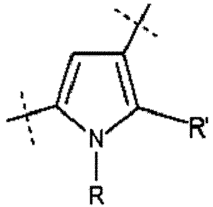
wherein each of R and R' is independently selected from the group consisting of H, C₁-C₄alkyl, linear or branched, C₁-C₄alkyl substituted with one or more halogens, and cyclopropyl;
and W is selected from the group consisting of O, S and NH,
the enantiomers, diastereoisomers, and mixtures thereof,
or a pharmaceutically acceptable salt, hydrate or solvate thereof,
with the proviso that Ar₁ and Ar₂ can not be at the same time both phenyl,
for use in the treatment of a disease which can be improved or prevented by an anti-heparanase activity.

2. The compound for the use according to claim 1 wherein said Ar₁ and Ar₂ are both 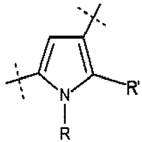 wherein R and R' have the meanings defined in claim 1.

3. The compound for the use according to claim 1 or 2 wherein said compound has the following formula (II):

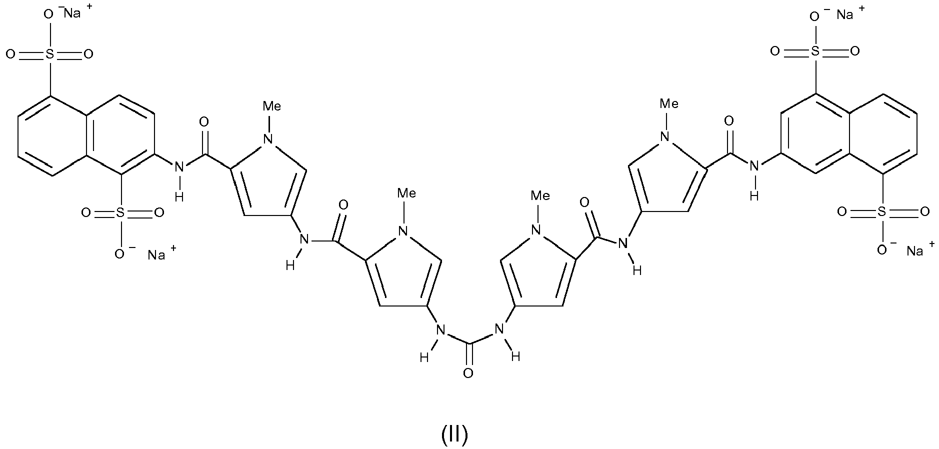

4. The compound for the use according to anyone of claims 1-3, wherein said disease is selected from the group consisting of diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging.

5. The compound for the use according to anyone of claims 1-3 , wherein said disease is selected from the group consisting of tumor, metastasis and multiple sclerosis.

6. The compound for the use according to claim 5 wherein said compound of formula (I) is not the compound of formula (II).

7. A pharmaceutical composition containing as active ingredient the compound of anyone of claims 1-6 and at least one pharmaceutically acceptable vehicle and/or excipient for use in the treatment of a disease which can be improved or prevented by an anti-heparanase activity.

8. The pharmaceutical composition for the use according to claim 7 wherein said composition further comprises one or more further active ingredients, preferably a chemotherapeutic agent.

9. A compound having the following formula (III) 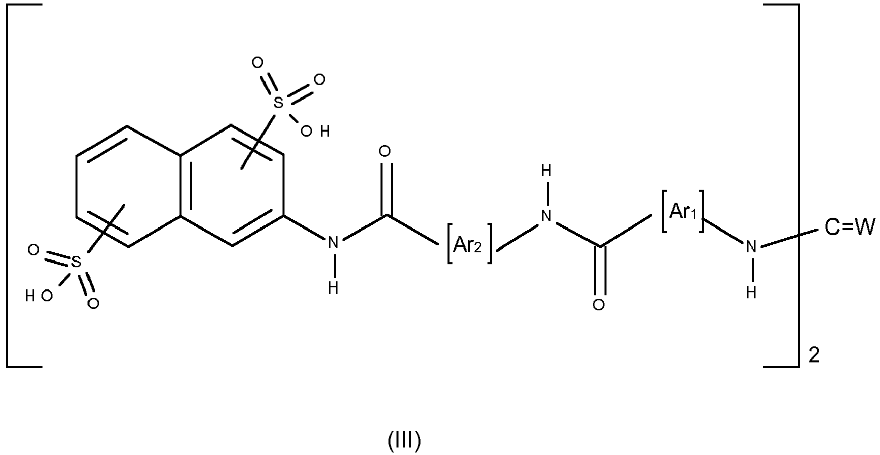 wherein
Ar₁ and Ar₂ are different from each other and selected from 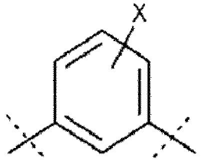
wherein X is selected from the group consisting of H, C₁-C₄alkyl, linear or branched, halogen and C₁-C₄alkyl substituted with one or more halogens,
and 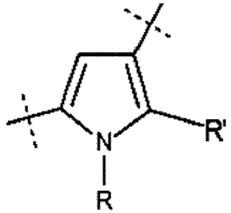
wherein each of R and R' is independently selected from the group consisting of H, C₁-C₄ alkyl, linear or branched, C₁-C₄alkyl substituted with one or more halogens, and cyclopropyl;
and W is selected from the group consisting of O, S and NH,
the enantiomers, diastereoisomers, and mixtures thereof,
or a pharmaceutically acceptable salt, hydrate or solvate thereof.

10. The compound according to claim 9 wherein W is O.

11. The compound according to anyone of claims 9-10 wherein Ar₁ or Ar₂ is 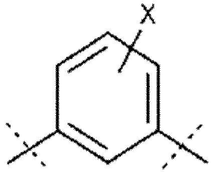 and X is selected from the group consisting of H, CH₃, F and CF₃.

12. The compound according to anyone of claims 9-11 wherein Ar₁ or Ar₂ is 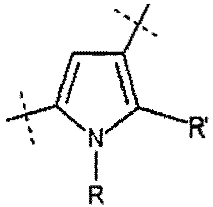 and R is selected from the group consisting of CH₃, CF₃ and cyclopropyl, and R' is selected from the group consisting of H, CH₃ and CF₃.

13. The compound according to anyone of claims 9-12, wherein W is O, Ar₁ is 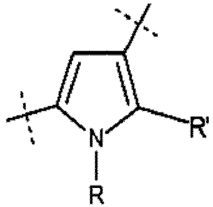 and Ar₂ is 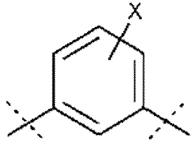 wherein X, R and R' have the meanings defined in the previous claims.

14. The compound according to claim 13 wherein X is H or CH₃, R is CH₃ and R' is H.

15. The compound according to anyone of claims 9-12, wherein W is O, Ar₁ is 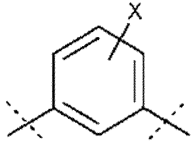 and Ar₂ is 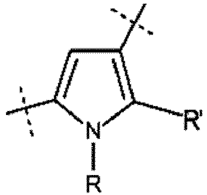 wherein X, R and R' have the meanings defined in the previous claims.

16. The compound according to claim 15 wherein X is H, F or CF₃, R is CH₃ or cyclopropyl and R' is H or CH₃.

17. The compound according to anyone of claims 9-16, which is in the form of a sodium salt.

18. The compound according to anyone of claims 9-17, which is selected from the following compounds:
- disodium 2,2'-{carbonylbis[imino(1-methyl-1*H*-pyrrole-4,2-diyl)carbonylimino-3,1-phenylenecarbonylimino]}dinaphthalene-1,5-disulfonate salt 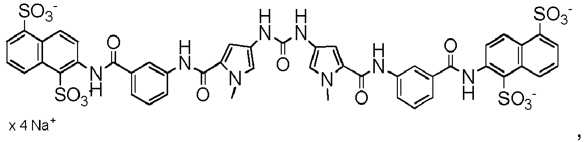
- disodium 2,2'-{carbonylbis[imino-3,1-phenylenecarbonylimino(1-methyl-1*H*-pyrrole-4,2-diyl)carbonylimino]}dinaphthalene-1,5-disulfonate salt 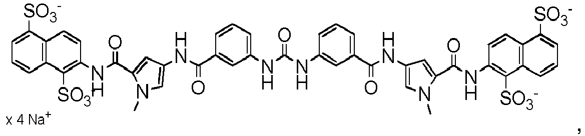
- disodium 2,2'-{carbonylbis[imino(1-methyl-1*H*-pyrrole-4,2-diyl)carbonylimino(4-methyl-3,1-phenylene)carbonylimino]}dinaphthalene-1,5-disulfonate salt 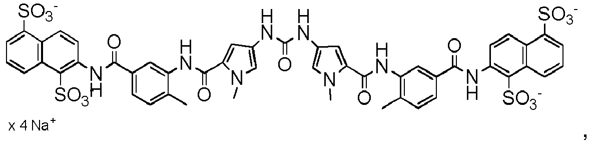
- disodium 2,2'-{carbonylbis[imino-3,1-phenylenecarbonylimino(1-cyclopropyl-1*H*-pyrrole-4,2-diyl)carbonylimino]}dinaphthalene-1,5-disulfonate salt 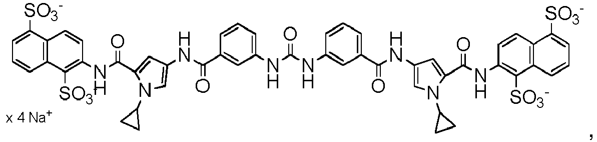
- disodium 2,2'-{carbonylbis[imino-3,1-phenylenecarbonylimino(1,5-dimethyl-1*H*-pyrrole-4,2-diyl)carbonylimino]}dinaphthalene-1,5-disulfonate salt 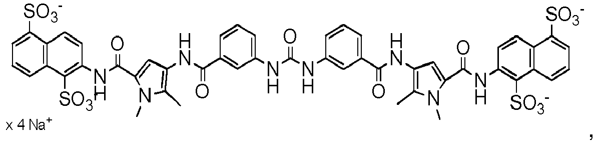
- disodium 2,2'-{carbonylbis[imino(4-fluoro-3,1-phenylene)carbonylimino(1-methyl-1*H*-pyrrole-4,2-diyl)carbonylimino]}dinaphthalene-1,5-disulfonate salt 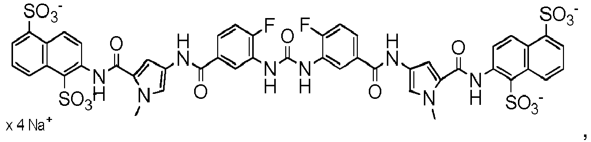 and
- disodium 2,2'-{carbonylbis[imino[5-(trifluoromethyl)-3,1-phenylene]carbonylimino(1-methyl-1H-pyrrole-4,2-diyl)carbonylimino]}dinaphthalene-1,5-disulfonate salt

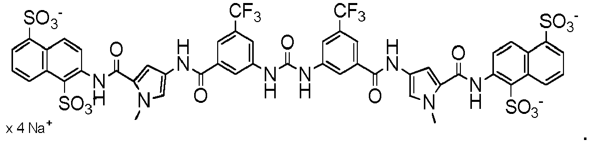

19. The compound of anyone of claims 9-18 for use as a medicament.

20. A pharmaceutical composition containing as active ingredient the compound of anyone of claims 9-18 and at least one pharmaceutically acceptable vehicle and/or excipient.

21. The pharmaceutical composition according to claim 20 wherein said composition further comprises one or more further active ingredients.

22. The pharmaceutical composition according to claim 21 wherein said further active ingredient is a chemotherapeutic agent.

23. The pharmaceutical composition according to claim 22 wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

24. The pharmaceutical composition according to claim 23 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
